# EUROPÄISCHE PATENTSCHRIFT

(11) **EP 3 378 857 B1**
(45) Veröffentlichungstag und Bekanntmachung des Hinweises auf die Patenterteilung: **24.03.2021**
(21) Anmeldenummer: 18170096.4
(22) Anmeldetag: 18.10.2013
(51) Int. Cl.: C07D 311/90, C07D 311/96, C07D 405/12, C09K 11/06, H01L 51/00

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 12.11.2012 EP 12007665
(43) Veröffentlichungstag der Anmeldung: 26.09.2018
(62) Teilanmeldung aus: 13779144.8
(73) Patentinhaber: Merck Patent GmbH, 64293 Darmstadt (DE)
(72) Erfinder: Mujica-Fernaud, Teresa, 64283 DARMSTADT (DE); Montenegro, Elvira, 69469 WEINHEIM (DE); Voges, Frank, 67098 BAD DUERKHEIM (DE); Kroeber, Jonas, 60311 FRANKFURT AM MAIN (DE); Stoessel, Philipp, 60389 FRANKFURT AM MAIN (DE)

(56) Entgegenhaltungen:
- CN-A- 101 440 082
- JP-A- 2009 191 232

## Beschreibung

Die vorliegende Anmeldung betrifft eine Verbindung mit Xanthen-Grundgerüst gemäß untenstehender Formel (I). Weiterhin betrifft die Anmeldung Verfahren zur Herstellung der Verbindung der Formel (I) und die Verwendung der Verbindung in einer elektronischen Vorrichtung.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter organische Elektrolumineszenzvorrichtungen (OLEDs) und andere elektronische Vorrichtungen verstanden, welche weiter unten aufgeführt sind.

Der Aufbau von OLEDs, in denen organische Halbleiter als funktionelle Materialien eingesetzt werden, ist beispielsweise in US 4539507, US 5151629, EP 0676461 und WO 98/27136 beschrieben. Allgemein werden unter der Bezeichnung OLED elektronische Vorrichtungen verstanden, welche organisches Material enthalten und unter Anlegen von elektrischer Spannung Licht emittieren.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Schichten mit lochtransportierender Funktion, wie beispielsweise lochinjizierende Schichten, Lochtransportschichten, Elektronenblockierschichten und emittierende Schichten.

Hierfür werden kontinuierlich neue Materialien mit lochtransportierenden Eigenschaften gesucht. Sie können in den genannten Schichten als Reinmaterialien, als Hauptkomponenten oder als Mindermengenkomponenten in Kombination mit weiteren Materialien eingesetzt werden.

In lochinjizierenden Schichten, Lochtransportschichten und Elektronenblockierschichten werden Materialien mit lochtransportierenden Eigenschaften typischerweise als Reinstoffe eingesetzt. Sie können jedoch in solchen Schichten auch in Mischung mit einem eindotierten weiteren Material eingesetzt werden. In emittierenden Schichten, insbesondere in phosphoreszierenden emittierenden Schichten, werden die Materialien mit lochtransportierenden Eigenschaften in vielen Fällen als eine der Hauptkomponenten der Schicht (Matrixmaterial, Hostmaterial) in Kombination mit weiteren Materialien, beispielsweise Emittermaterialien eingesetzt.

Es ist im Stand der Technik bekannt, Triarylamine als Materialien mit lochtransportierenden Eigenschaften in den oben genannten Schichten einzusetzen. Diese können Mono-Triarylamine darstellen, wie beispielsweise in JP 1995/053955, WO 2006/123667 und JP 2010/222268 beschrieben, oder Bis- oder andere Oligoamine darstellen, wie beispielsweise in US 7504163 oder US 2005/0184657 beschrieben. Bekannte Beispiele für Triarylamin-Verbindungen als Materialien mit lochtransportierenden Eigenschaften für OLEDs sind unter anderem Tris-p-biphenyl-amin, N,N'-Di-1-Naphthyl-N,N'-diphenyl-1,1'-biphenyl-4,4'-diamin (NPB) und 4,4',4"-Tris-(3-methylphenylphenylamino)triphenylamin (MTDATA).

Im Stand der Technik bekannt ist die Verwendung von Xanthen-Verbindungen, die mit Arylgruppen substituiert sind, als Matrixmaterialien für phosphoreszierende Emitter in OLEDs (US 7014925).

Weiterhin im Stand der Technik bekannt sind Verbindungen, die ein Xanthen-Grundgerüst aufweisen, und die Arylamino-Gruppen tragen. Beispielsweise werden in JP 2009-191232 Xanthen-Arylamin-Verbindungen als emittierende Verbindungen in OLEDs offenbart. Weiterhin werden in CN 101440082 Xanthen-Diamin-Verbindungen offenbart, die in OLEDs als Funktionsmaterialien in der emittierenden Schicht eingesetzt werden.

Überraschend wurde nun gefunden, dass mit einer Xanthen-Verbindung nach Formel (I), wie untenstehend definiert, welche eine einzige Arylaminogruppe aufweist, hervorragende Leistungsdaten in elektronischen Vorrichtungen erzielt werden können.

Gegenstand der Erfindung ist somit eine Verbindung einer Formel (I) 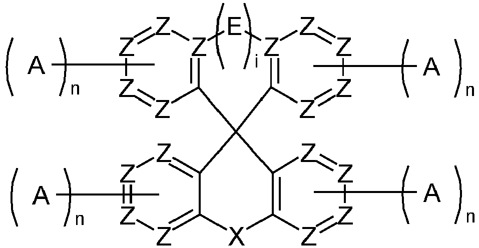 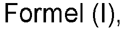 wobei die auftretenden Variablen definiert sind wie in Anspruch 1.

Es gelten die folgenden Definitionen und Erläuterungen:
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, 0 und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, 0 und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

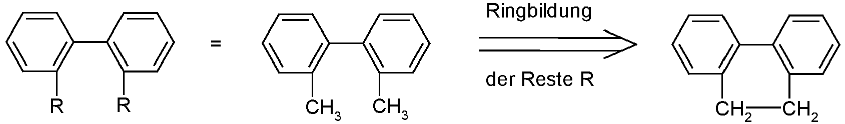

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

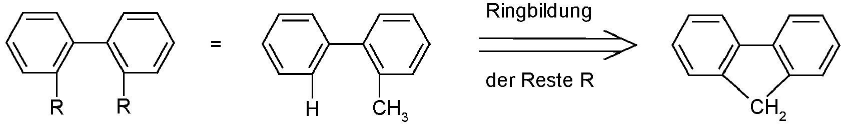

Betreffend die Indices n ind i gilt, dass wenn ein Index gleich Null ist, die betreffende Gruppe nicht vorhanden ist. Beispielsweise ist für i=0 die Gruppe E nicht vorhanden, so dass die beiden aromatischen Sechsringe nicht über eine Einfachbindung zu einem Fluoren-Ringsystem verbunden sind.

Betreffend die Reste R¹ bis R³ gelten folgende allgemein bevorzugte Definitionen:
R¹ ist bevorzugt bei jedem Auftreten gleich oder verschieden H, D, F, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, -R³C=CR³-, Si(R³)₂, C=O, C=NR³, -NR³-, -O-, -S-, -C(=O)O- oder - C(=O)NR³- ersetzt sein können, oder ein aromatisches oder heteroaromatisches Ringsystem mit 5 bis 20 aromatischen Ringatomen, das jeweils mit einem oder mehreren Resten R³ substituiert sein kann, wobei zwei oder mehr Reste R¹ miteinander verknüpft sein können und einen Ring bilden können.

R² ist bevorzugt bei jedem Auftreten gleich oder verschieden H, D, F, CN, Si(R³)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, -R³C=CR³-, Si(R³)₂, C=O, C=NR³, -O-, -S-, -C(=O)O- oder -C(=O)NR³-ersetzt sein können, wobei zwei oder mehr Reste R² miteinander verknüpft sein können und einen Ring bilden können.

R³ ist bevorzugt bei jedem Auftreten gleich oder verschieden H, D, F, CN, Si(R⁴)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, C=NR⁴, -NR⁴-, -O-, -S-, -C(=O)O- oder - C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

In Formel (I) ist X bevorzugt gleich 0. Diese bevorzugte Ausführungsform ist bevorzugt mit allen bevorzugten Ausführungsformen der Gruppen und Indices in Formel (I) zu kombinieren, insbesondere mit den bevorzugten Ausführungsformen von A und den Resten R¹ bis R³.

Bevorzugt umfasst die Verbindung der Formel (I) keine kondensierte Arylgruppe mit mehr als 14 aromatischen Ringatomen, besonders bevorzugt keine kondensierte Arylgruppe mit mehr als 10 aromatischen Ringatomen.

Bevorzugt ist L¹ bei jedem Auftreten gleich oder verschieden ein aromatisches oder heteroaromatisches Ringsystem mit 6 bis 18 aromatischen Ringatomen, welches mit einem oder mehreren Resten R¹ substituiert sein kann. Ganz besonders bevorzugt ist L¹ bei jedem Auftreten gleich oder verschieden Phenyl, Biphenyl, Naphthyl, Terphenyl, Fluorenyl, Spirobifluoren, Indenofluorenyl, Carbazol, Dibenzofuran, oder Dibenzothiophen, die jeweils mit einem oder mehreren Resten R¹ substituiert sein können.

Weiterhin bevorzugt ist in Formel (A-II) k gleich 0 oder 1, besonders bevorzugt gleich 0.

Weiterhin bevorzugt ist in Formel (A-II) Ar¹ bei jedem Auftreten gleich oder verschieden ein aromatisches oder heteroaromatisches Ringsystem mit 6 bis 24 aromatischen Ringatomen, das mit einem oder mehreren Resten R¹ substituiert sein kann. Ganz besonders bevorzugt sind darunter Phenyl, Biphenyl, Naphthyl, Terphenyl, Fluorenyl, Spirobifluoren, Indenofluorenyl, Carbazol, Dibenzofuran, und Dibenzothiophen, die mit einem oder mehreren Resten R¹ substituiert sein können.

Besonders bevorzugte Gruppen A entsprechen den Formeln (A-II-19) und (A-II-20):

| | |
|---|---|
| 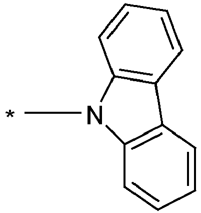 | 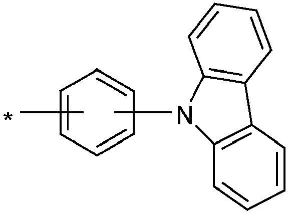 |
| Formel (A-II-19) | Formel (A-II-20) |

wobei die Gruppen an allen freien Positionen mit einem oder mehreren Resten R¹, wie oben definiert, substituiert sein können. Bevorzugt ist, dass Reste R¹ dabei definiert sind gemäß ihren bevorzugten Ausführungsformen.

In untenstehender Formel (I-num) sind die möglichen Bindungspositionen der Gruppen A nummeriert angegeben.

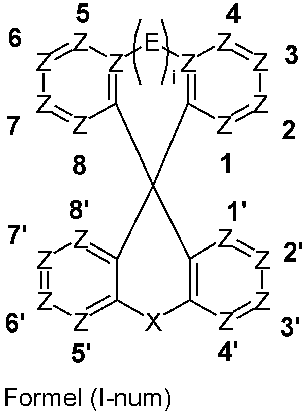

Entsprechend obenstehender Nummerierung ist es bevorzugt, dass die Gruppe A in Position 2, 4, 2', 4', 5, 7, 5' oder 7' gebunden ist. Besonders bevorzugt ist die Gruppe A in Position 2, 2', 7 oder 7' gebunden. Dabei kann i gleich 0 oder 1 sein. Weiterhin ist dabei bevorzugt X gleich 0.

Bevorzugte Ausführungsformen der Verbindungen der Formel (I) entsprechen somit einer der Formeln (I-1) bis (I-8): 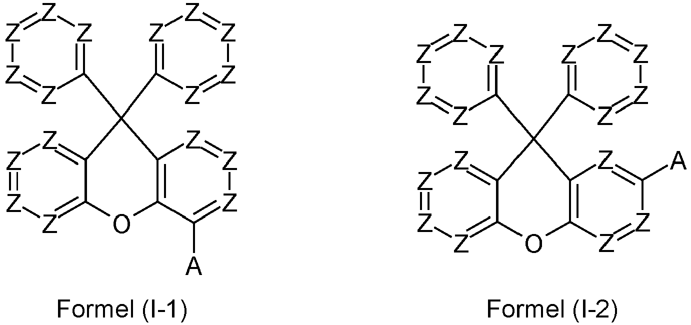 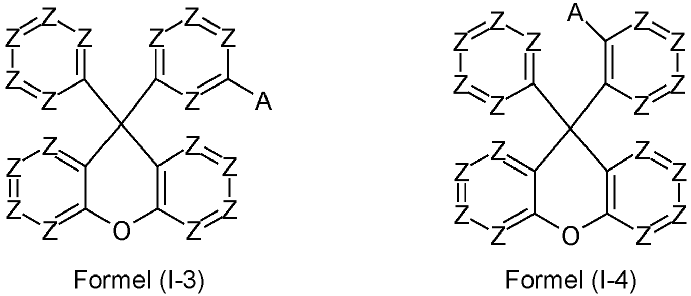 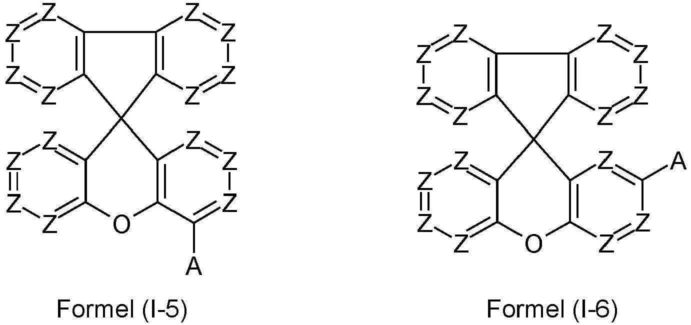 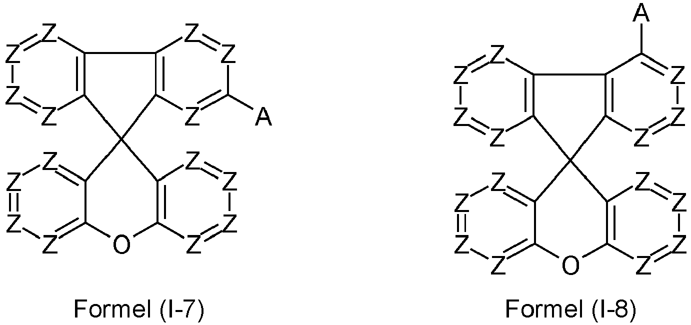 wobei die auftretenden Symbole wie oben definiert sind.

Es gelten für die Verbindungen der Formeln (I-1) bis (I-8) die oben angegebenen bevorzugten Ausführungsformen der variablen Gruppen. Besonders bevorzugte Ausführungsformen von Verbindungen der Formel (I) sind die in der folgenden Tabelle angegebenen Strukturen.

Sie setzen sich zusammen aus einem Grundkörper gewählt aus Grundkörpern der Formeln (I-1) bis (I-8) und aus Gruppen A gewählt aus den bevorzugten Ausführungsformen der Formeln (A-II-19) und (A-II-20).

| | Grundkörper | Gruppe A |
|---|---|---|
| (I-1-19) | (I-1) | (A-II-19) |
| (I-1-20) | " | (A-II-20) |
| (I-2-19) | (I-2) | (A-II-19) |
| (I-2-20) | " | (A-II-20) |
| (I-3-19) | (I-3) | (A-II-19) |
| (I-3-20) | " | (A-II-20) |
| (I-4-19) | (I-4) | (A-II-19) |
| (I-4-20) | " | (A-II-20) |
| (I-5-19) | (I-5) | (A-II-19) |
| (I-5-20) | " | (A-II-20) |
| (I-6-19) | (I-6) | (A-II-19) |
| (I-6-20) | " | (A-II-20) |
| (I-7-19) | (I-7) | (A-II-19) |
| (I-7-20) | " | (A-II-20) |
| (I-8-19) | (I-8) | (A-II-19) |
| (I-8-20) | " | (A-II-20) |

Für die tabellarisch aufgeführten besonders bevorzugten Ausführungsformen gelten die oben angegebenen bevorzugten Ausführungsformen der variablen Gruppen, inbesondere der von Z und R¹ bis R³.

Explizite Beispiele für Verbindungen der Formel (I) sind in der folgenden Tabelle abgebildet. Nicht anspruchsgemäße Verbindungen sind dabei mit # markiert:

| | | |
|---|---|---|
| 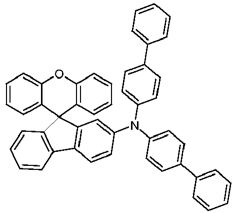 | 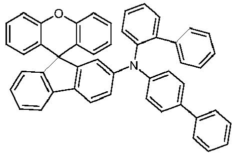 | 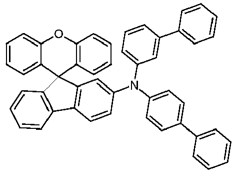 |
| 1# | 2# | 3# |
| 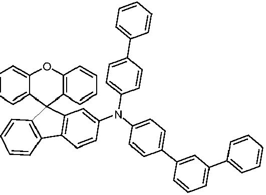 | 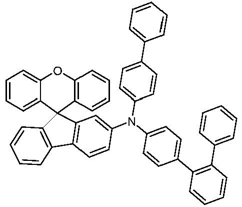 | 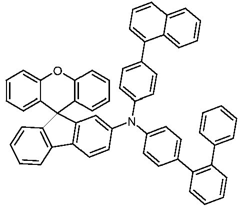 |
| 4# | 5# | 6# |
| 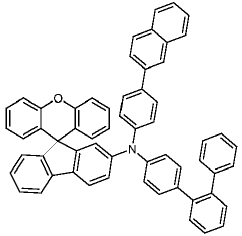 | 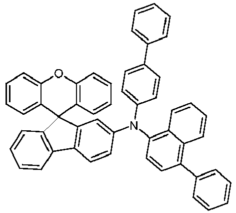 | 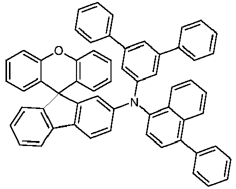 |
| 7# | 8# | 9# |
| 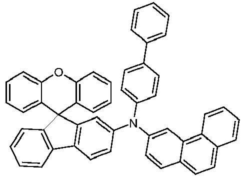 | 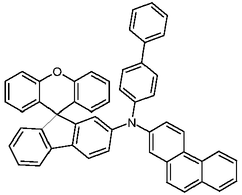 | 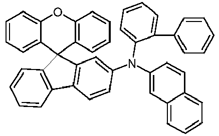 |
| 10# | 11# | 12# |
| 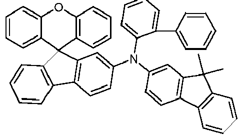 | 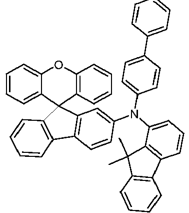 | 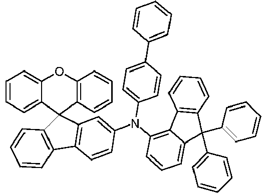 |
| 13# | 14# | 15# |
| 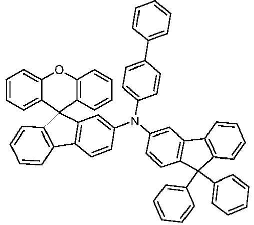 | 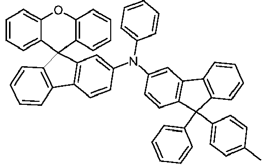 | 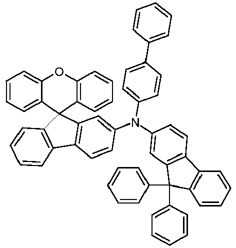 |
| 15# | 17# | 18# |
| 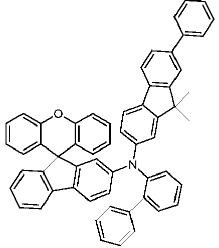 | 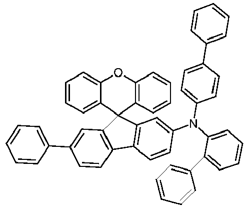 | 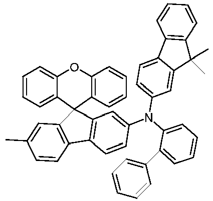 |
| 19# | 20# | 21# |
| 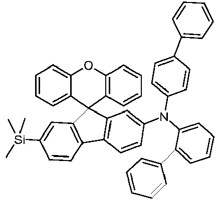 | 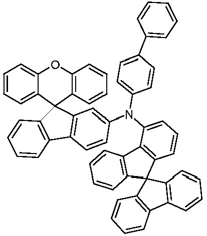 | 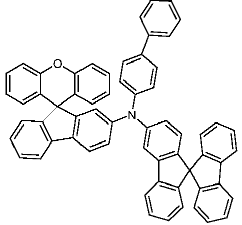 |
| 22# | 23# | 24# |
| 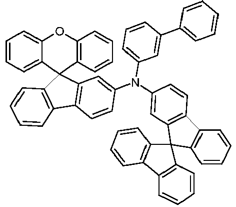 | 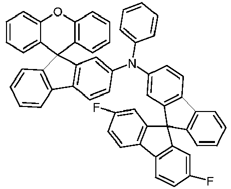 | 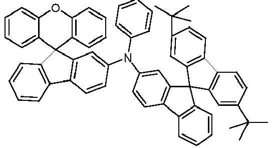 |
| 25# | 26# | 27# |
| 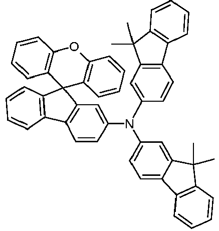 | 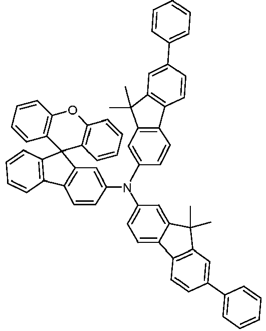 | 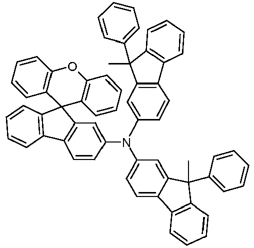 |
| 28# | 29# | 30# |
| 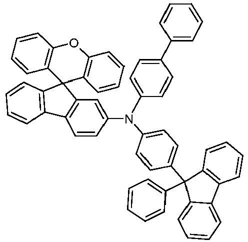 | 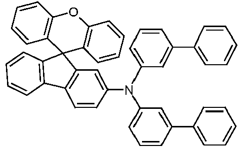 | 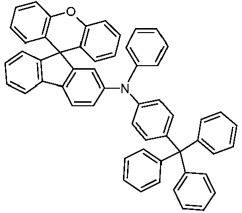 |
| 31# | 32# | 33# |
| 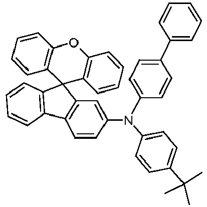 | 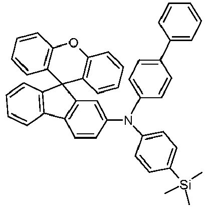 | 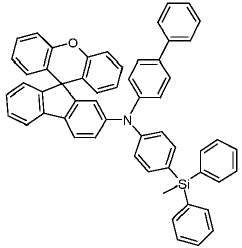 |
| 34# | 35# | 36# |
| 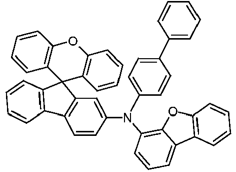 | 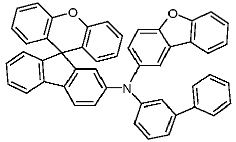 | 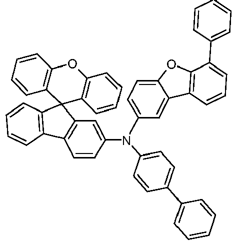 |
| 37# | 38# | 39# |
| 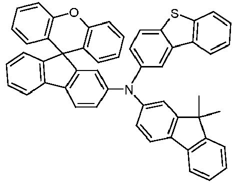 | 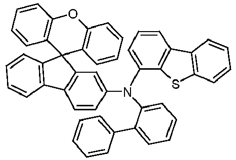 | 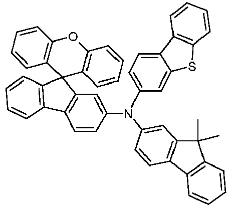 |
| 40# | 41# | 42# |
| 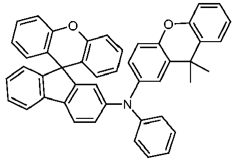 | 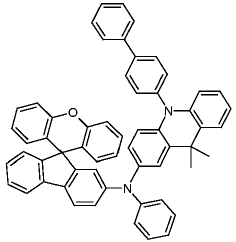 | 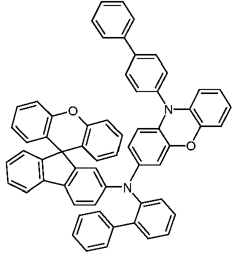 |
| 43# | 44# | 45# |
| 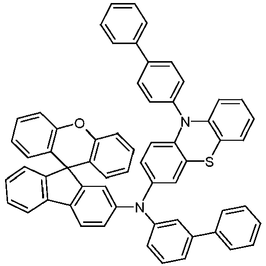 | 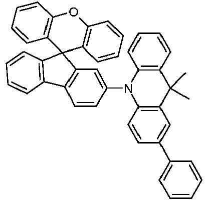 | 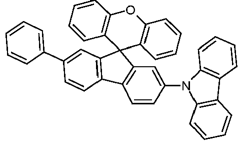 |
| 46# | 47# | 48 |
| 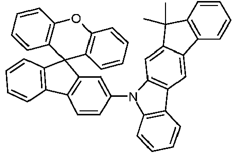 | 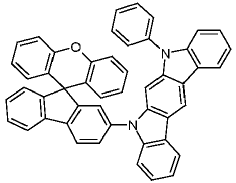 | 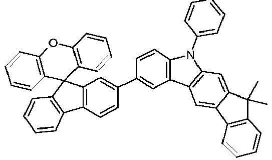 |
| 49 | 50 | 51# |
| 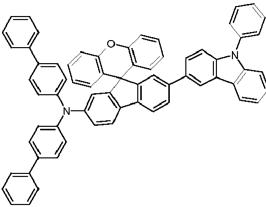 | 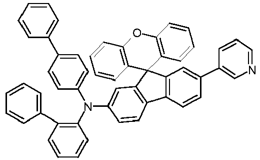 | 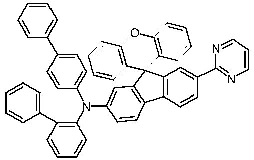 |
| 52# | 53# | 54# |
| 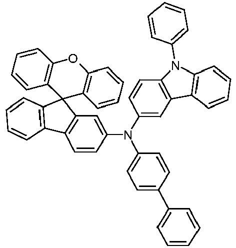 | 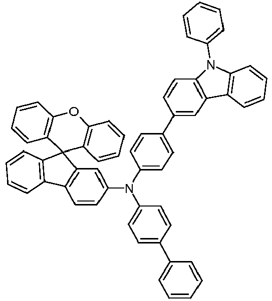 | 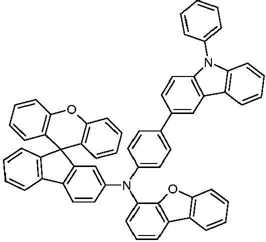 |
| 55# | 56# | 57# |
| 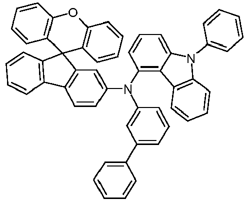 | 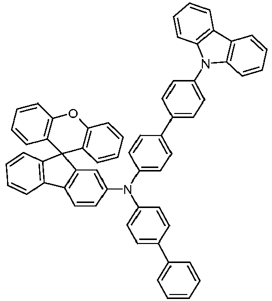 | 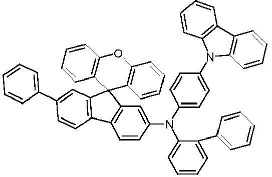 |
| 58# | 59# | 60# |
| 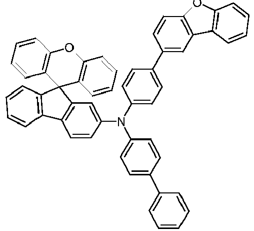 | 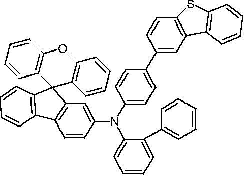 | 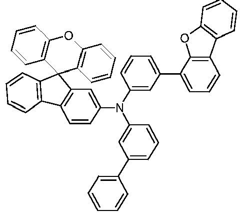 |
| 61# | 62# | 63# |
| 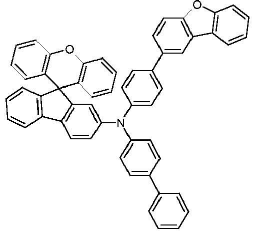 | 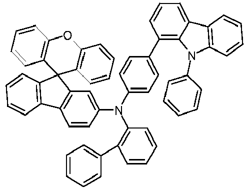 | 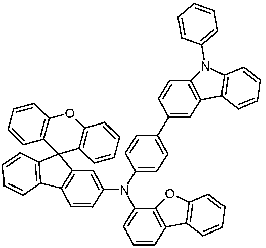 |
| 64# | 65# | 66# |
| 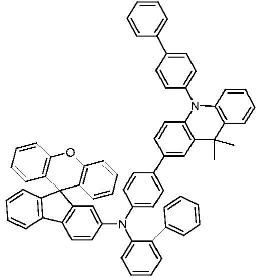 | 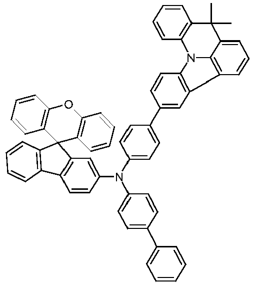 | 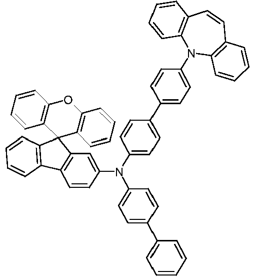 |
| 67# | 68# | 69# |
| 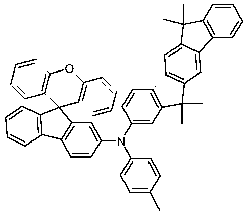 | 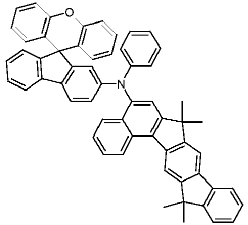 | 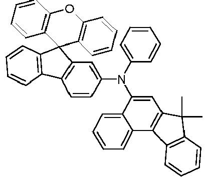 |
| 70# | 71# | 72# |
| 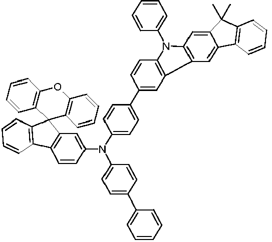 | 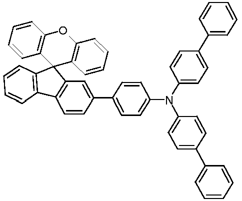 | 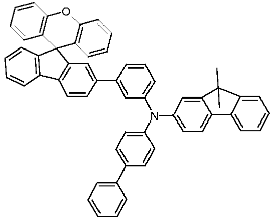 |
| 73# | 74# | 75# |
| 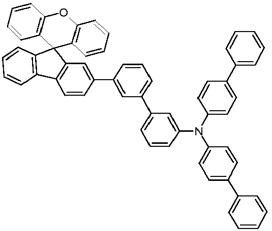 | 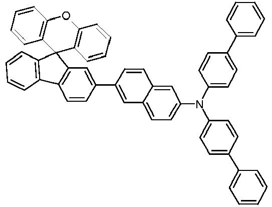 | 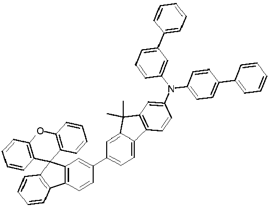 |
| 76# | 77# | 78# |
| 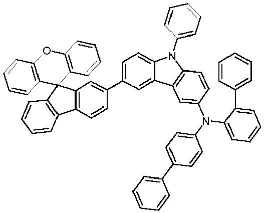 | 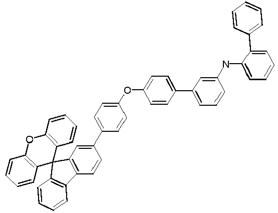 | 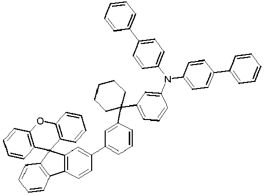 |
| 79# | 80# | 81# |
| 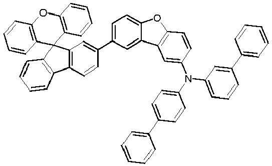 | 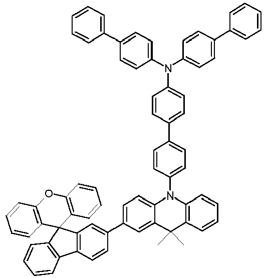 | 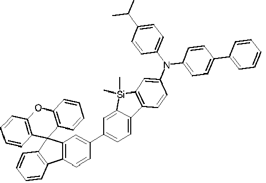 |
| 82# | 83# | 84# |
| 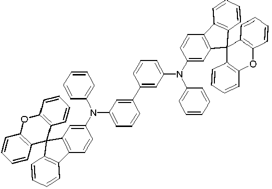 | 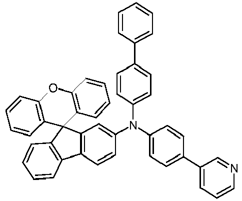 | 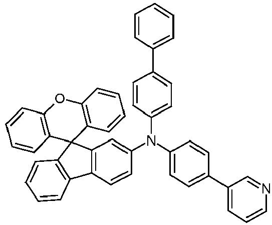 |
| 85# | 86# | 87# |
| 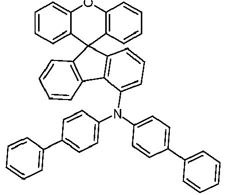 | 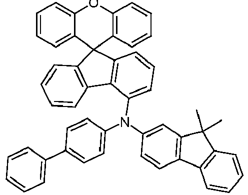 | 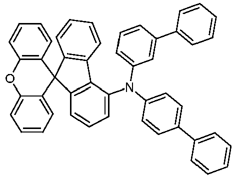 |
| 88# | 89# | 90# |
| 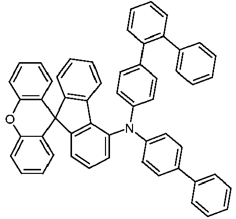 | 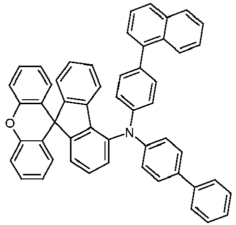 | 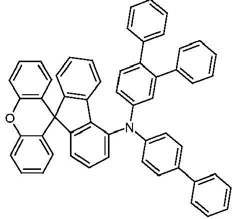 |
| 91# | 92# | 93# |
| 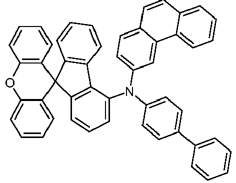 | 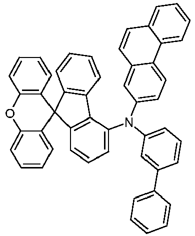 | 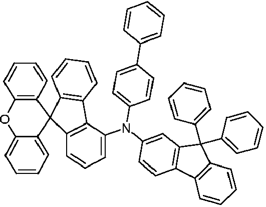 |
| 94# | 95# | 96# |
| 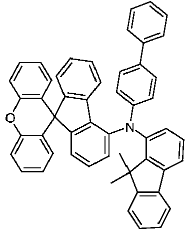 | 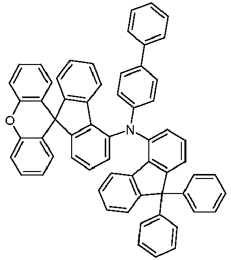 | 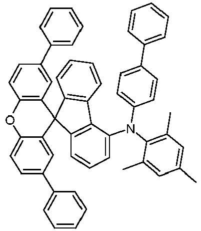 |
| 97# | 98# | 99# |
| 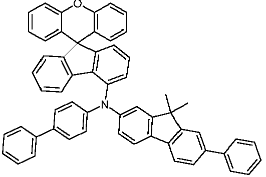 | 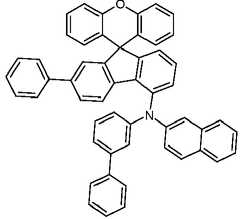 | 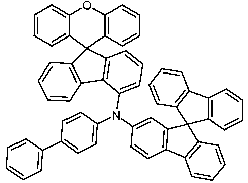 |
| 100# | 101# | 102# |
| 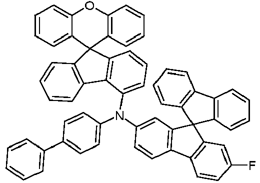 | 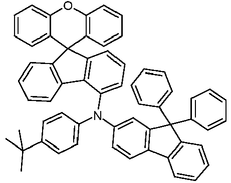 | 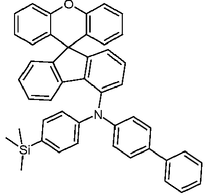 |
| 103# | 104# | 105# |
| 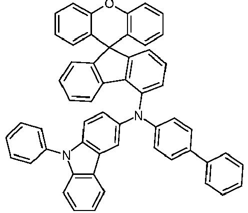 | 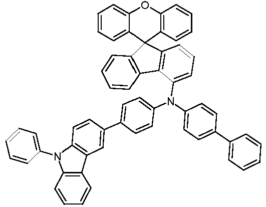 | 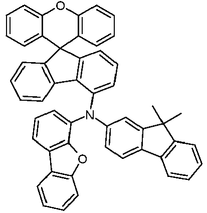 |
| 106# | 107# | 108# |
| 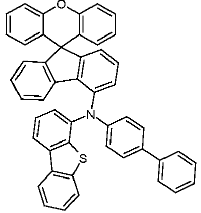 | 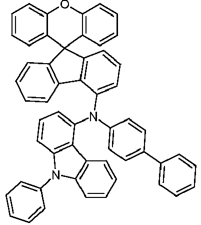 | 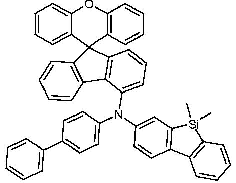 |
| 109# | 110# | 111# |
| 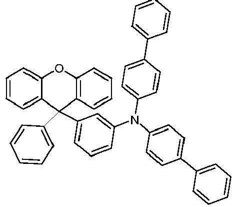 | 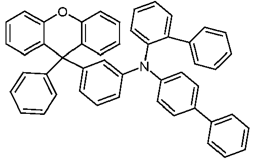 | 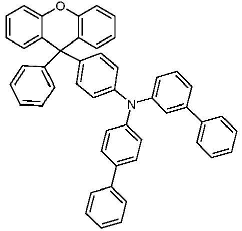 |
| 112# | 113# | 114# |
| 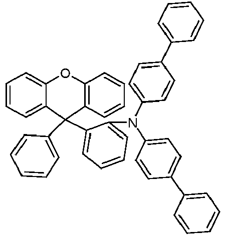 | 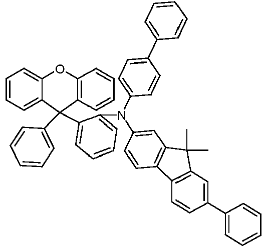 | 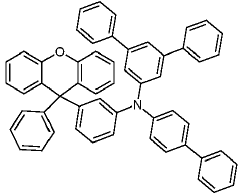 |
| 115# | 116# | 117# |
| 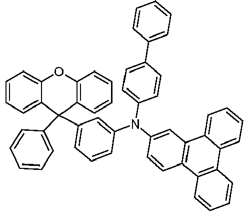 | 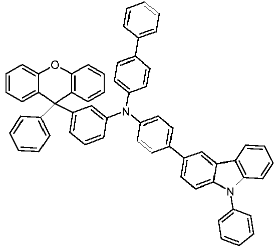 | 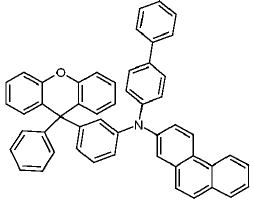 |
| 118# | 119# | 120# |
| 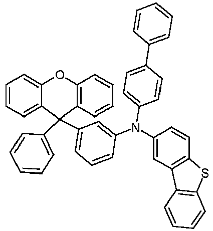 | 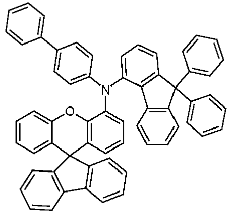 | 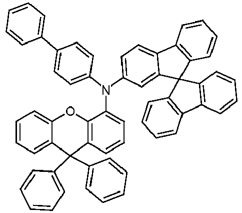 |
| 121# | 122# | 123# |
| 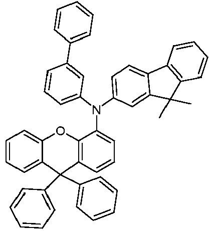 | 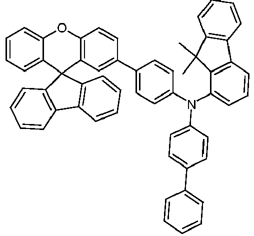 | 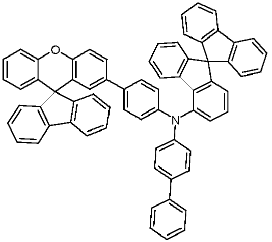 |
| 124# | 125# | 126# |
| 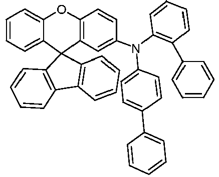 | 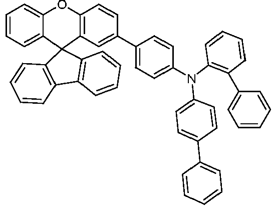 | 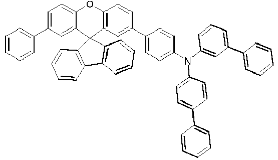 |
| 127# | 128# | 129# |
| 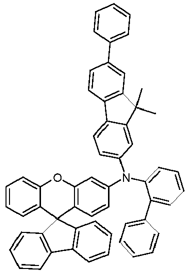 | 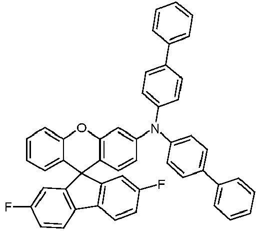 | 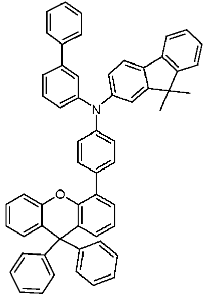 |
| 130# | 131# | 132# |
| 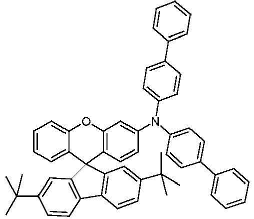 | | |
| 133# | | |

Die Synthese der erfindungsgemäßen Verbindungen kann gemäß im Stand der Technik bekannten Verfahren und Reaktionstypen, beispielsweise Halogenierung, metallorganische Addition, Buchwald-Kupplung und Suzuki-Kupplung erfolgen.

Schemata 1 bis 3 zeigen mögliche Synthesewege zur Herstellung der erfindungsgemäßen Verbindungen. Sie dienen zur Erläuterung der Erfindung für den Fachmann und sind nicht einschränkend auszulegen. Der Fachmann kann im Rahmen seines allgemeinen Fachwissens die gezeigten Synthesewege abwandeln, oder völlig andere Wege entwickeln, falls dies vorteilhafter erscheint.

Schema 1 zeigt einen bevorzugten Syntheseweg zur Herstellung von erfindungsgemäßen Verbindungen, die ein Diaryl-Xanthen-Grundgerüst aufweisen (In Formel (I) Index i=0).

Die Metallierung eines 2-Halogen-substituierten Diarylethers (A) mit reaktiven Metallen (z.B. Magnesium nach Grignard) oder mit Organo-Lithium-Verbindungen und anschließende Addition an mono-halogeniertes Benzophenon (B) und anschließende säurekatalysierte Cyclisierung des intermediären Alkoholats führt zu den entsprechenden halogensubstituierten Xanthenen (C). Die so entstandenen Halogenide (C) können anschließend nach dem Fachmann geläufigen Methoden (C-C-Kuplung wie Suzuki-, Negishi-, Yamamoto-, Grignard-Cross-, Stille-, Heck-Kupplung, etc.; C-N-Kupplung wie Buchwald- oder Ullmann-Kupplung weiter zu erfindungsgemäßen Verbindungen D und E umgesetzt werden.

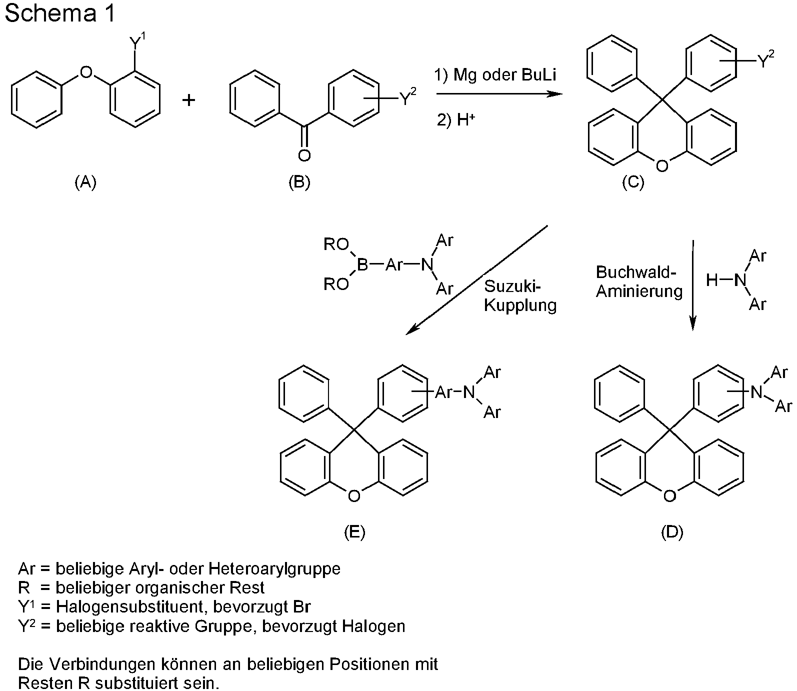

Schema 2 zeigt, wie analog zu den in Schema 1 gezeigten Diaryl-Xanthenen die entsprechenden Spirobifluoren-Xanthen-Verbindungen hergestellt werden können (Verbindungen H und I). Dazu wird im ersten Schritt, der Addition an die Carbonylverbindung und Cyclisierung, anstelle des Benzophenon-Derivats ein Fluorenon-Derivat (Verbindung F) eingesetzt.

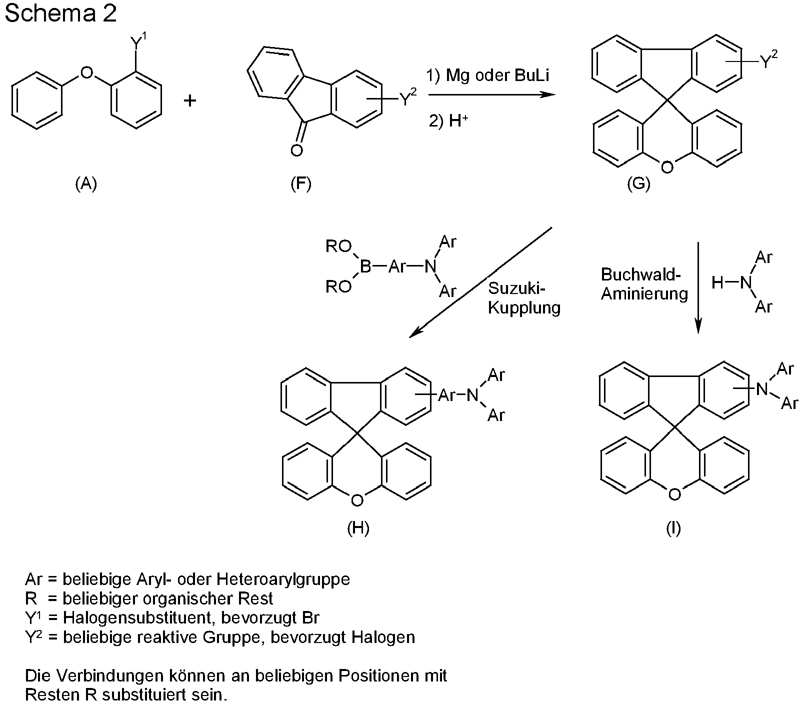

Ein anderer bevorzugter Syntheseweg zur Herstellung der erfindungsgemäßen Verbindungen ist in Schema 3 gezeigt. Damit können insbesondere Spirofluoren-Xanthen-Derivate hergestellt werden.

Der Syntheseweg umfasst die Metallierung einer 2-Halogen-substituierten Diaryl-Verbindung (K) mit reaktiven Metallen (z.B. Magnesium nach Grignard) oder mit Organo-Lithium-Verbindungen. Anschließend findet eine Addition an mono-halogeniertes Xanthenon (J) und eine säurekatalysierte Cyclisierung des intermediären Alkoholats statt. Damit werden die entsprechenden halogensubstituierten Spirofluoren-Xanthene (L) erhalten.

Die so entstandenen Halogenide (L) können anschließend nach dem Fachmann geläufigen Methoden (C-C-Kuplung wie Suzuki-, Negishi-, Yamamoto-, Grignard-Cross-, Stille-, Heck-Kupplung, etc.; C-N-Kupplung wie Buchwald- oder Ullmann-Kupplung weiter zu erfindungsgemäßen Verbindungen (M) und (N) umgesetzt werden.

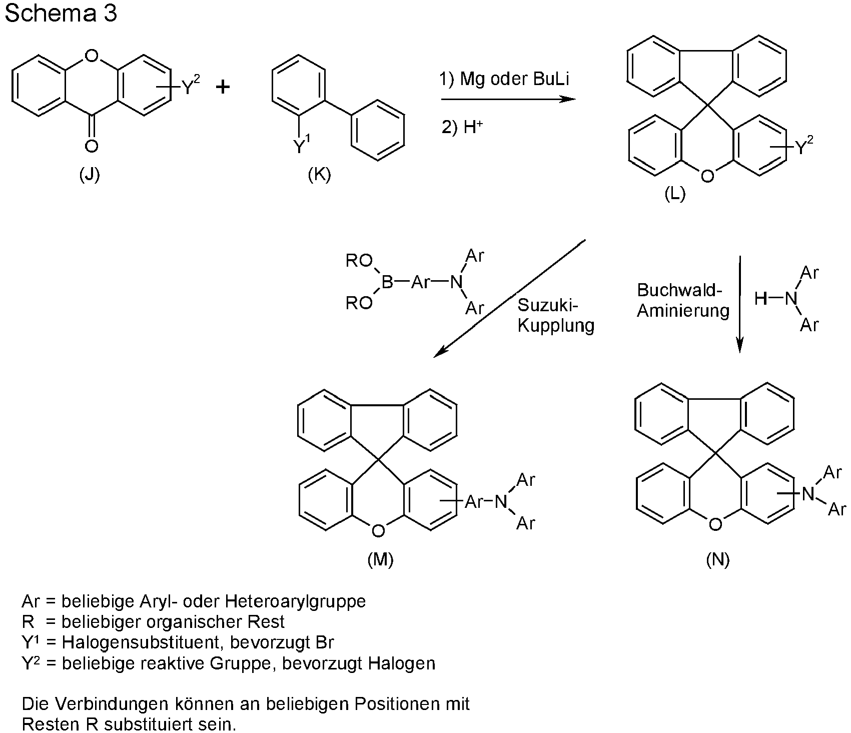

Synthesewege für die Ausgangsverbindungen (beispielsweise (A), (B), (F) und (K)), welche in der Synthese der erfindungsgemäßen Verbindungen eingesetzt werden, sind dem Fachmann bekannt. Die Kupplungsreaktionen stellen dabei bevorzugt Buchwald-Kupplungen und Suzuki-Kupplungen dar.

Die erhaltenen Verbindungen können im Anschluss an die oben gezeigten Syntheseschritte wahlweise weiter umgesetzt und funktionalisiert werden, falls dies erforderlich ist, um zu den gewünschten erfindungsgemäßen Verbindungen zu gelangen.

Weiterer Gegenstand der Erfindung ist somit ein Verfahren zur Herstellung einer Verbindung der Formel (I), dadurch gekennzeichnet, dass der Grundkörper durch Addition eines metallorganischen Nukleophils an eine Carbonylgruppe hergestellt wird.

Die Carbonylgruppe ist dabei bevorzugt eine Diarylcarbonylgruppe. Das metallorganische Nukleophil ist dabei bevorzugt ein Diarylether oder ein Diarylthioether, besonders bevorzugt ein Diarylether.

Bevorzugt wird in einem weiteren Schritt durch Kupplungsreaktion, besonders bevorzugt Buchwald-Kupplung oder Suzuki-Kupplung, die Arylaminogruppe eingeführt.

Detailliert beschriebene Syntheseverfahren, in denen unter anderem exakte Reaktionsbedingungen angegeben sind, sind in den Ausführungsbeispielen aufgeführt. Sie ergänzen die oben angegebenen allgemeinen Verfahren um konkrete Beispiele.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der organischen Elektrolumineszenzvorrichtung ist bevorzugt die folgende:
Anode-Lochinjektionsschicht-Lochtransportschicht-emittierende Schicht-Elektronentransportschicht-Elektroneninjektionsschicht-Kathode.
Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Alternativ und/oder zusätzlich können die erfindungsgemäßen Verbindungen auch in der Lochtransportschicht oder in einer anderen Schicht vorhanden sein.
Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (I) in einer elektronischen Vorrichtung enthaltend einen oder mehrere phosphoreszierende Emitter eingesetzt wird. Dabei kann die Verbindung in unterschiedlichen Schichten, bevorzugt in einer Lochtransportschicht, einer Elektronenblockierschicht, einer Lochinjektionsschicht oder in einer emittierenden Schicht, enthalten sein.

Vom Begriff phosphoreszierende Emitter sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen.

Explizite Beispiele für geeignete phosphoreszierende Emitterverbindungen können einer später folgenden Tabelle mit allgemein bevorzugten phosphoreszierenden Emittern entnommen werden.

Die Verbindung gemäß Formel (I) kann aber auch erfindungsgemäß in einer elektronischen Vorrichtung enthaltend einen oder mehrere fluoreszierende Emitter eingesetzt werden.

In einer bevorzugten Ausführungsform der Erfindung werden die Verbindungen gemäß Formel (I) als Lochtransportmaterial eingesetzt. Die Verbindungen werden dann bevorzugt in einer Lochtransportschicht, einer Elektronenblockierschicht oder einer Lochinjektionsschicht eingesetzt.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

In einer weiteren Ausführungsform der vorliegenden Erfindung werden die Verbindungen der Formel (I) als Matrixmaterial in Kombination mit einem oder mehreren Emittern, vorzugsweise phosphoreszierenden Emittern, eingesetzt.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Emitters zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Emitter enthalten.

In einer weiteren bevorzugten Ausführungsform der Erfindung werden die Verbindungen gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Emitter umfassen, bevorzugt einen oder mehrere phosphoreszierende Emitter. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Emitter oder den bevorzugten Matrixmaterialien für fluoreszierende Emitter, je nachdem welche Art von Emitterverbindung im mixed-Matrix-System eingesetzt wird.

Bevorzugte phosphoreszierende Emitter zur Verwendung in Mixed-Matrix-Systemen sind die obenstehend und in die in einer folgenden Tabelle aufgeführten phosphoreszierenden Emitter.

In einer nochmals weiteren bevorzugten Ausführungsform der Erfindung wird die Verbindung der Formel (I) als fluoreszierender Emitter in einer emittierenden Schicht eingesetzt.

Wenn die erfindungsgemäße Verbindung als emittierendes Material in einer emittierenden Schicht eingesetzt wird, wird sie bevorzugt in Kombination mit einem oder mehreren Matrixmaterialien eingesetzt. Bevorzugte Matrixmaterialien zur Verwendung in Kombination mit der Verbindung der Formel (I) als Emitter sind in folgenden Abschnitten angegeben.

Im Folgenden werden in den erfindungsgemäßen Vorrichtungen bevorzugt eingesetzte Materialien aufgeführt, geordnet nach ihrer Verwendung und Funktion.

Explizite Beispiele für phosphoreszierende Emitter sind in der folgenden Tabelle aufgeführt.

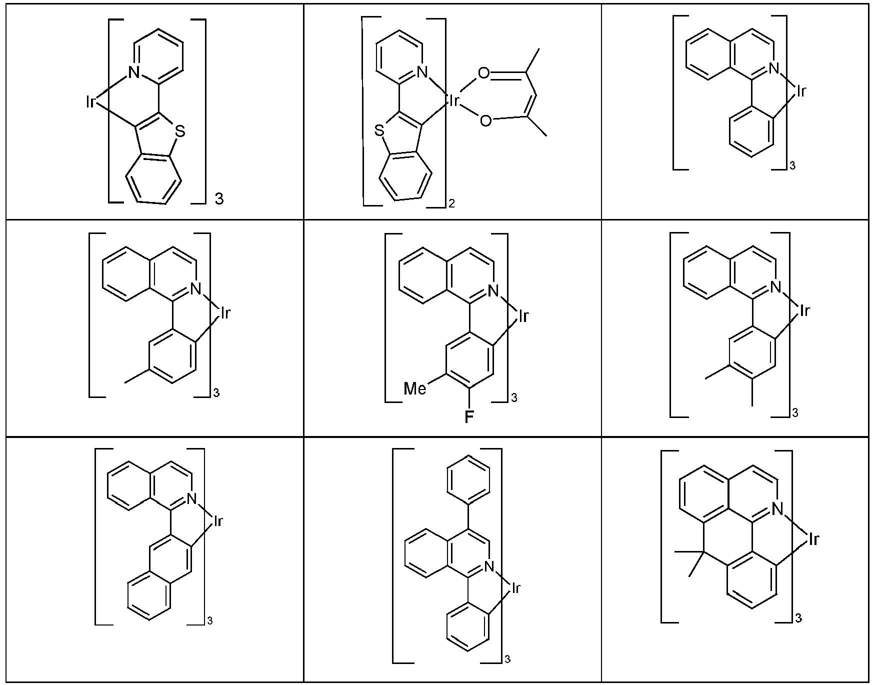

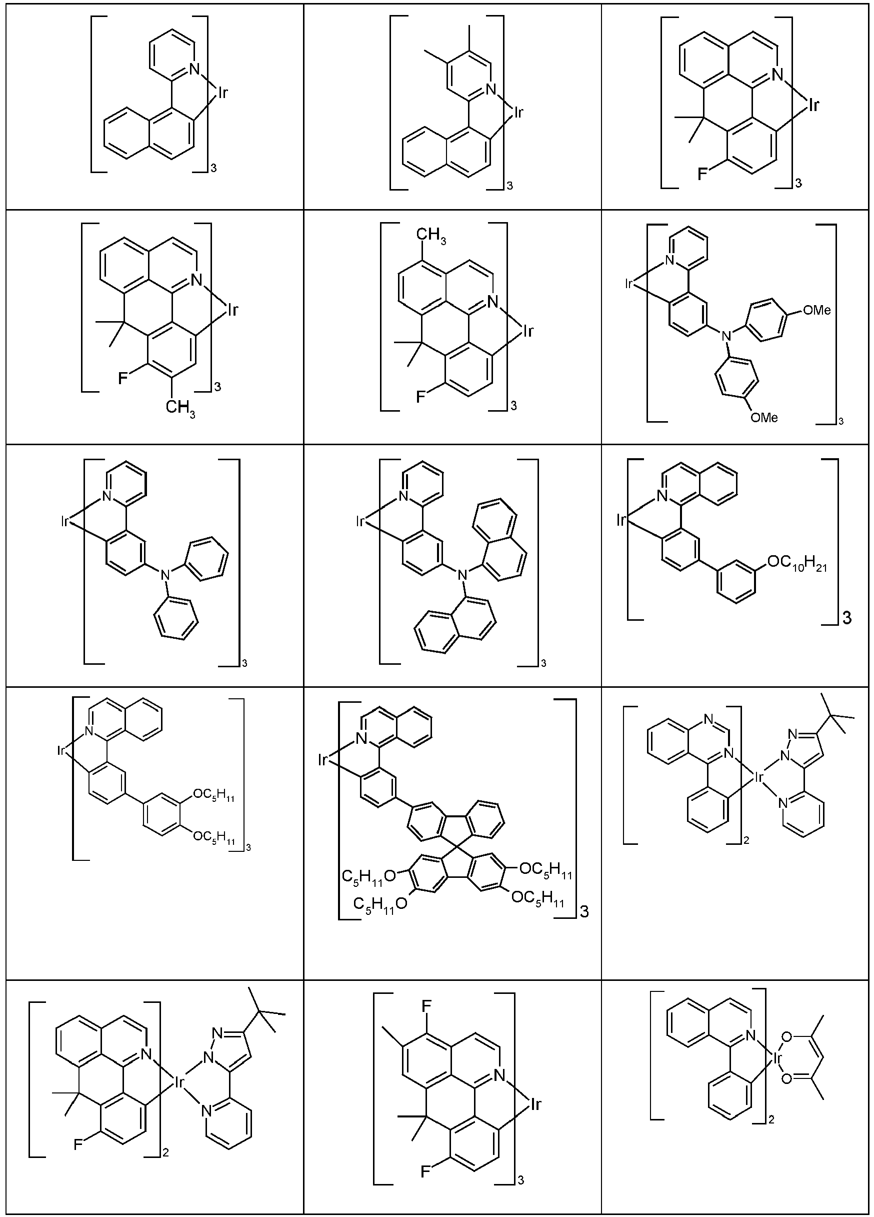

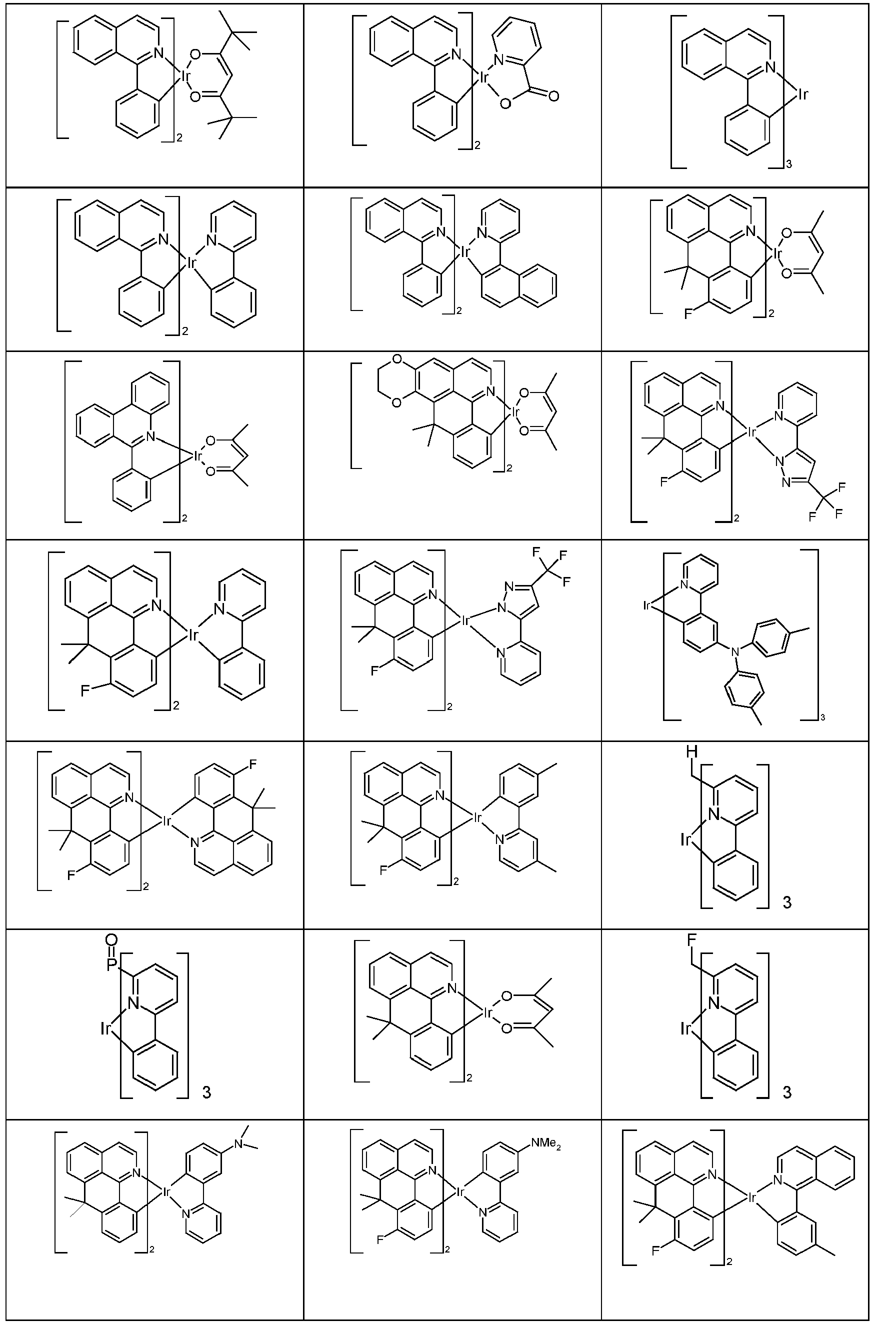

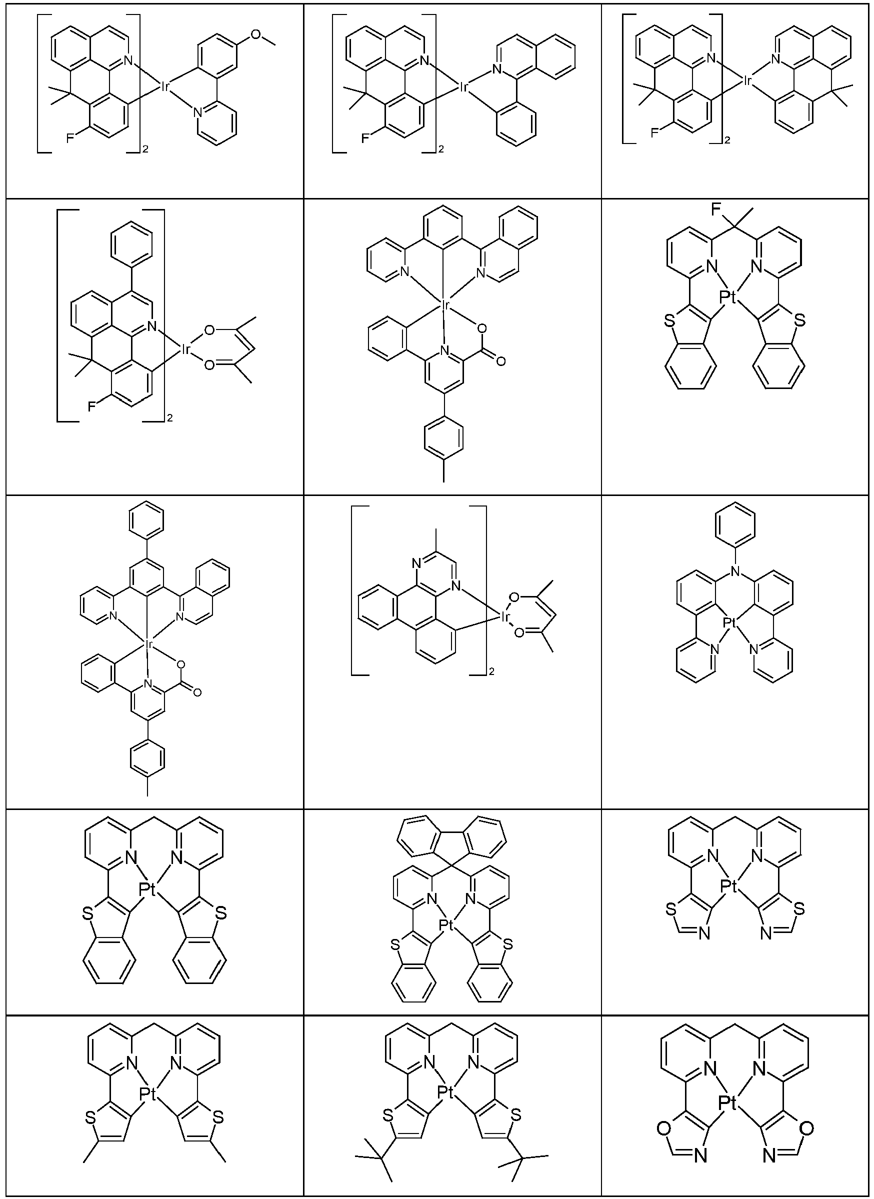

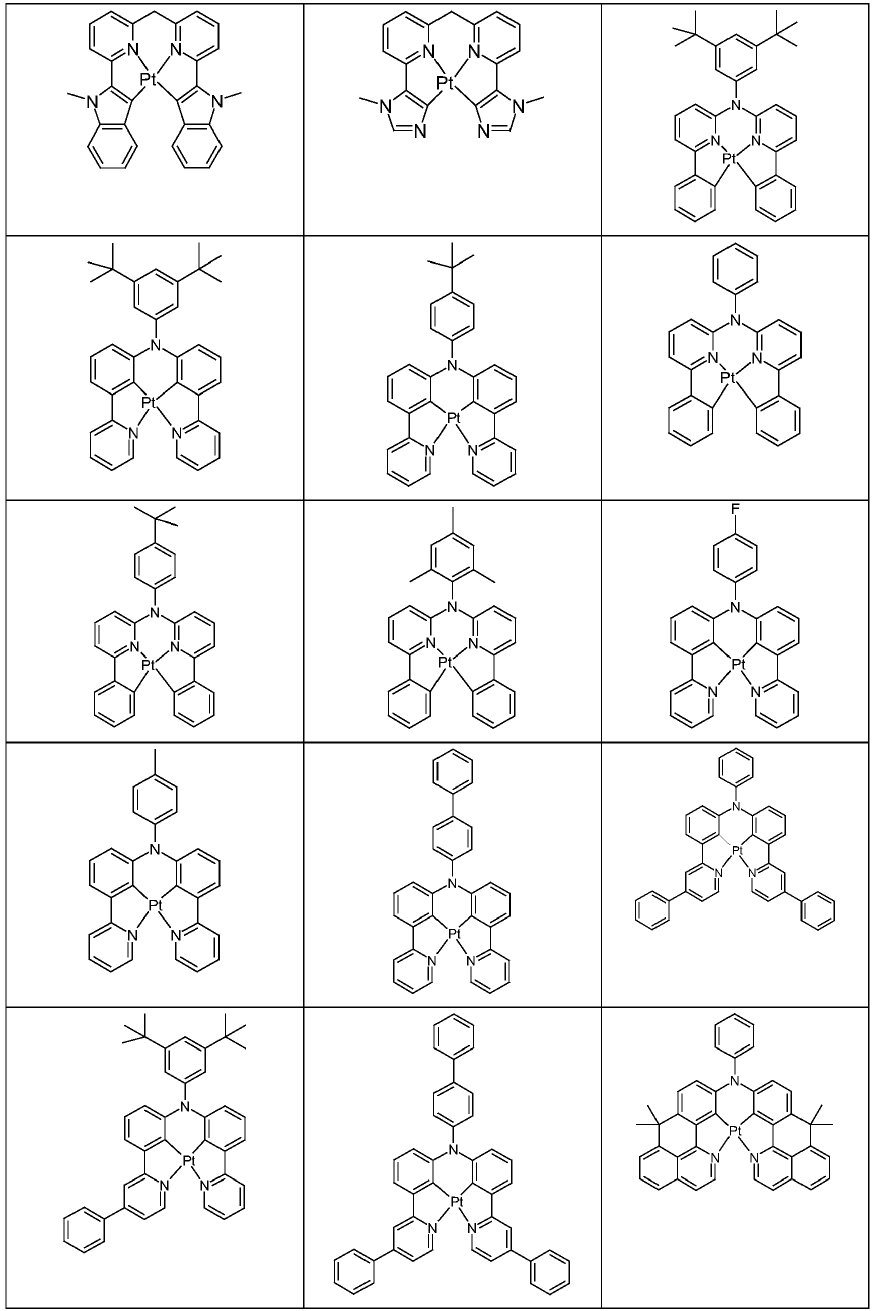

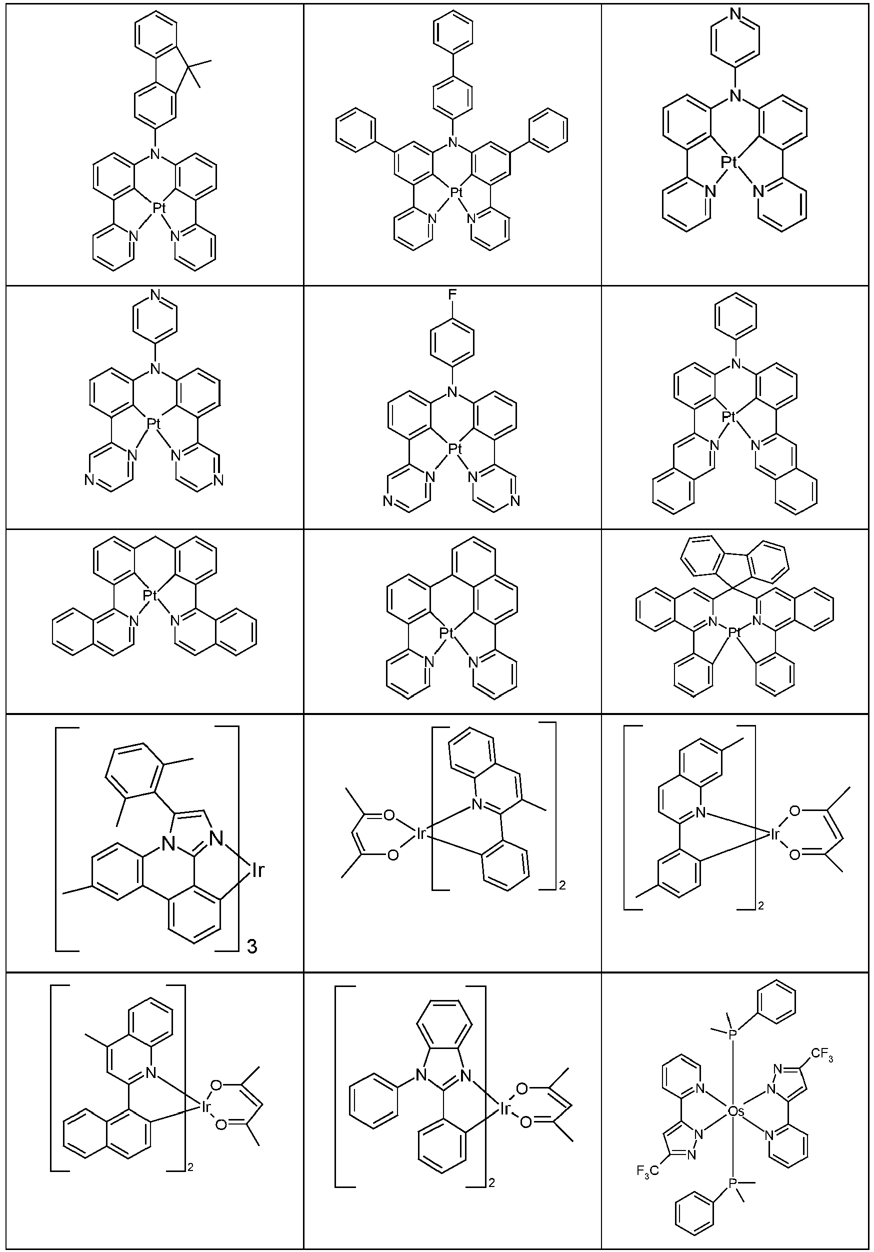

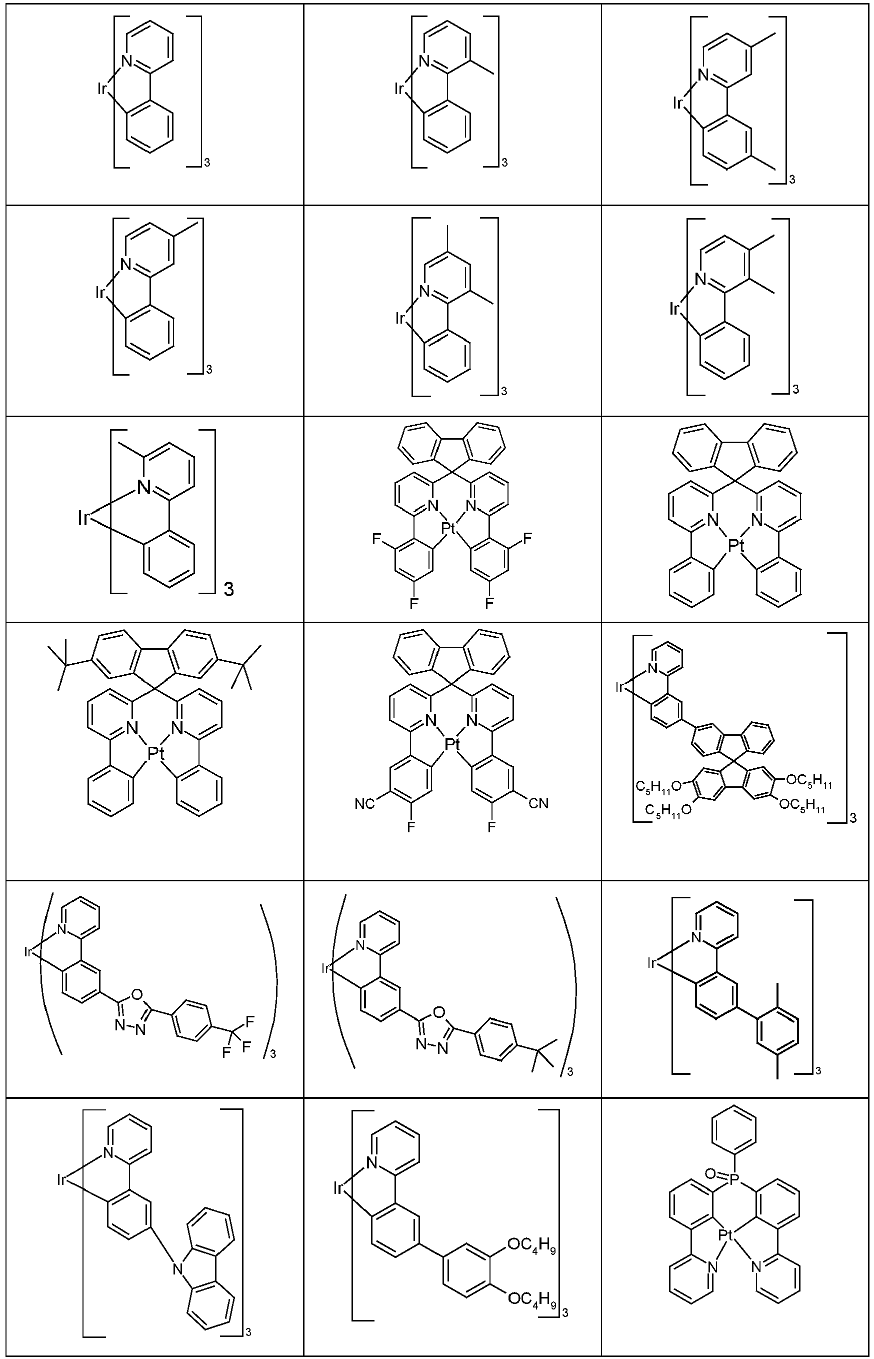

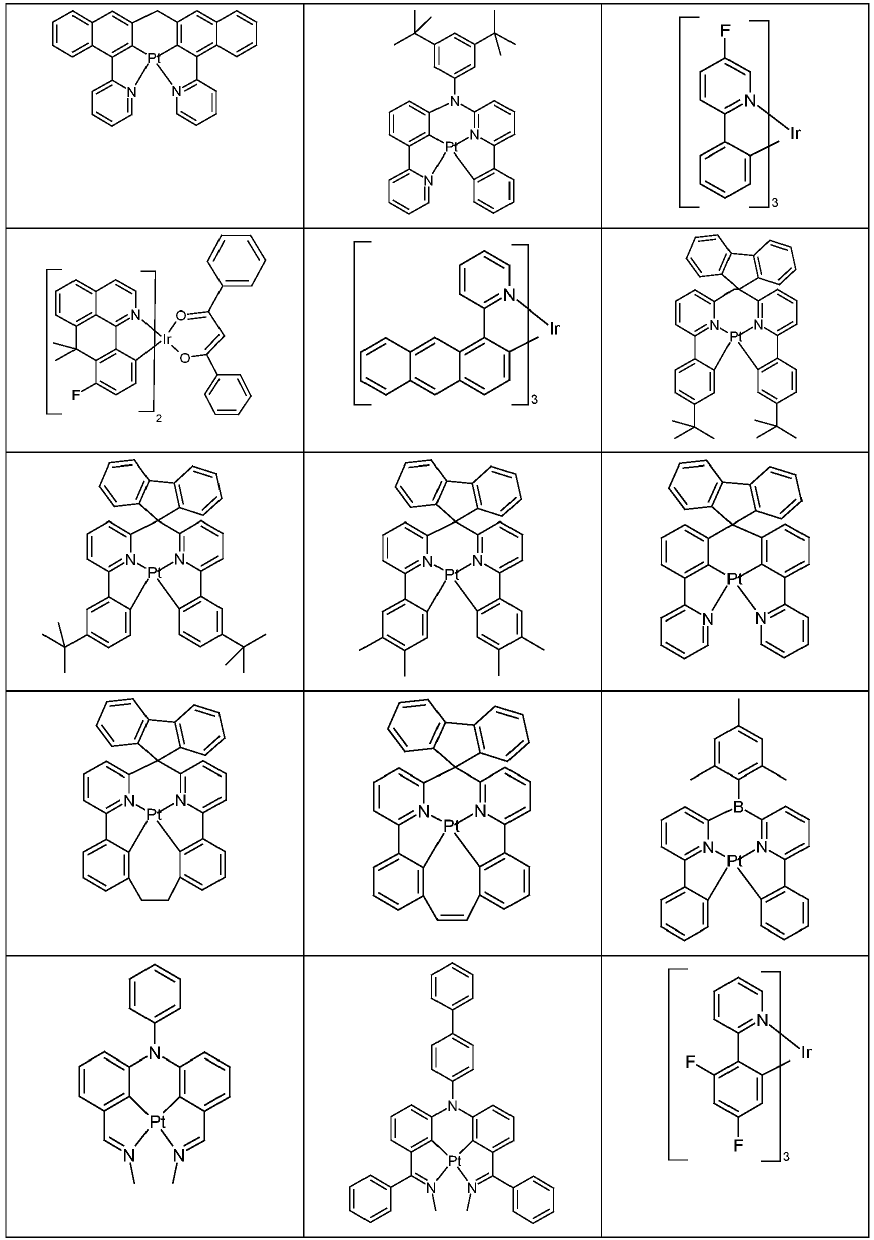

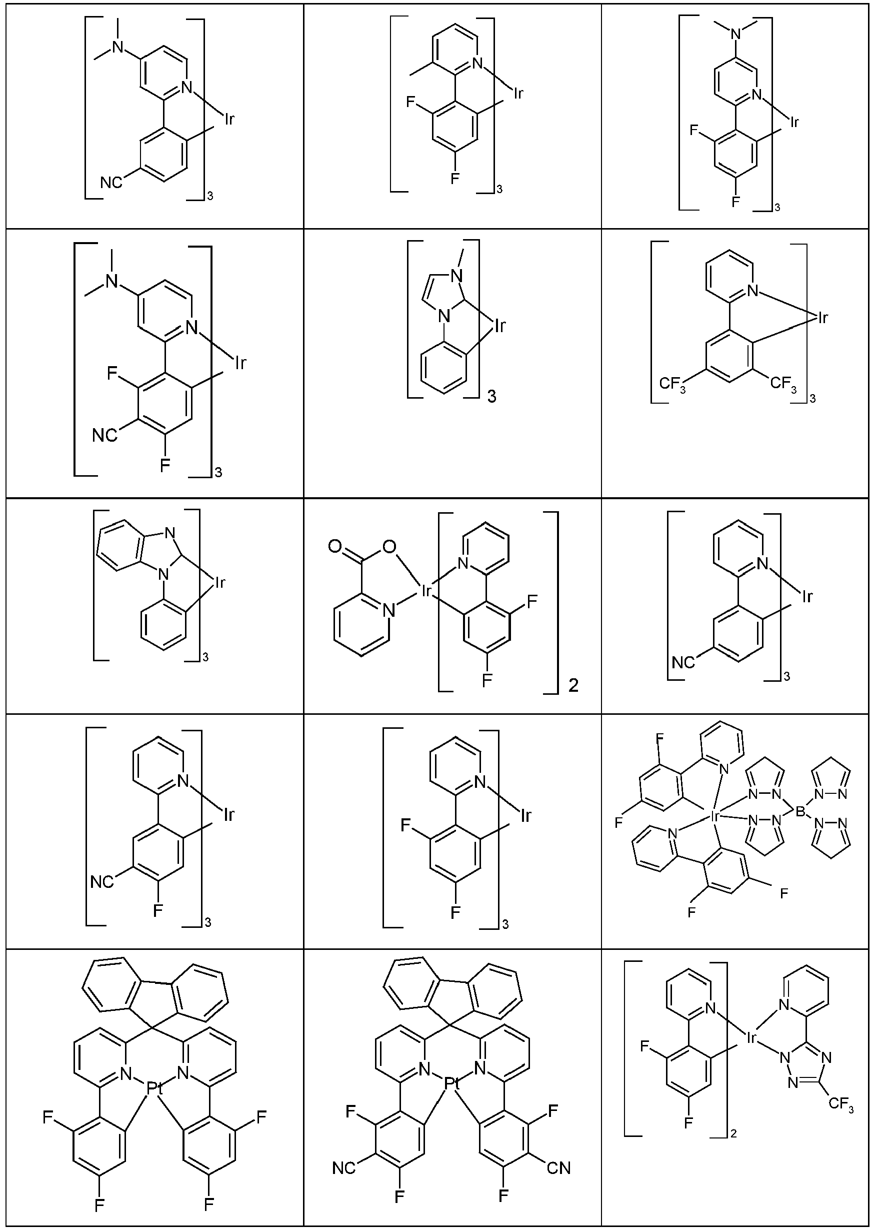

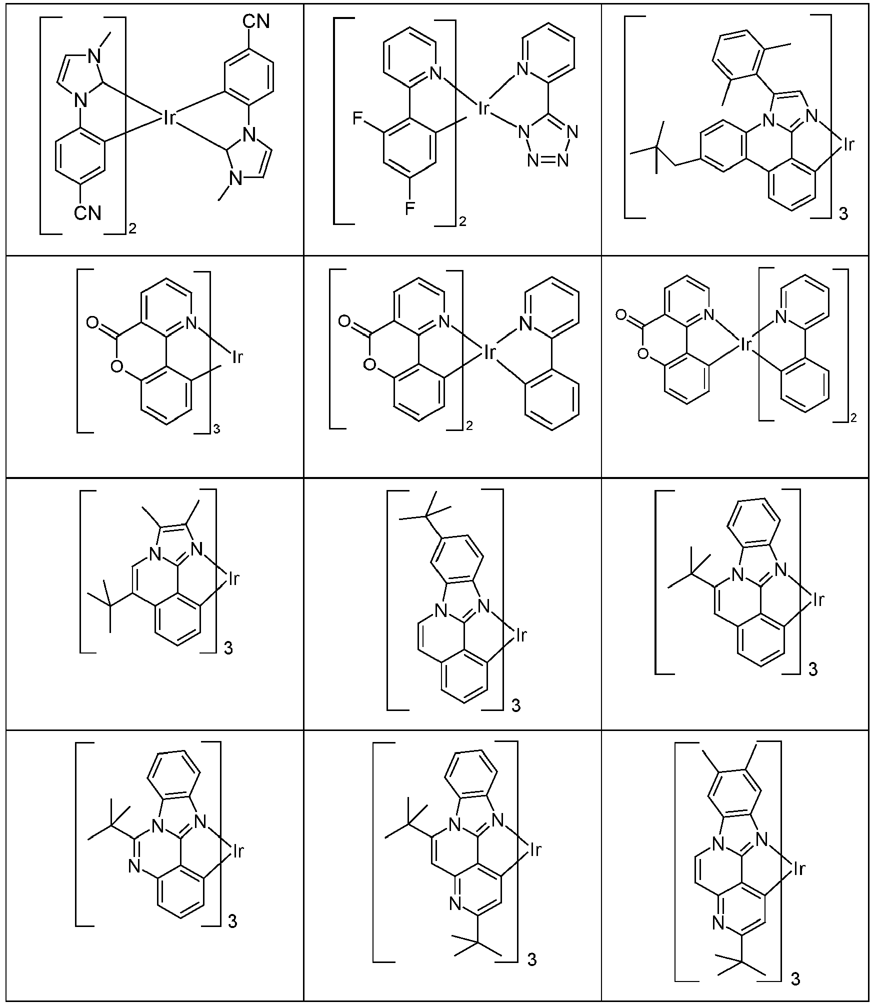

Bevorzugte fluoreszierende Emitter sind neben den erfindungsgemäßen Verbindungen ausgewählt aus der Klasse der Arylamine. Unter einem Arylamin bzw. einem aromatischen Amin wird dabei eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Emitter sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und der noch nicht offengelegten EP 12004426.8 offenbarten Pyren-Arylamine. Ebenfalls bevorzugt sind die in der noch nicht offengelegten EP 12006239.3 offenbarten Benzoindenofluoren-Amine.

Als Matrixmaterialien, bevorzugt für fluoreszierende Emitter, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Besonders bevorzugt als Matrixmaterialien für fluoreszierende Emitter sind die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate. Bevorzugt als Matrixmaterialien für fluoreszierende Emitter sind die in EP 1749809, EP 1905754 und US 2012/0187826 offenbarten Pyren-Verbindungen.

Bevorzugte Matrixmaterialien für phosphoreszierende Emitter sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729 und DiazaphospholDerivate, z. B. gemäß WO 2010/054730.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001). Auch die erfindungsgemäßen Verbindungen können als Lochtransportmaterialien verwendet werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

Nicht anspruchsgemäße Beispiele sind im Folgenden mit# gekennzeichnet.

### A) Synthesebeispiele

### Beispiel 1-1 #:

### Synthese der erfindungsgemäßen Verbindung 1-1

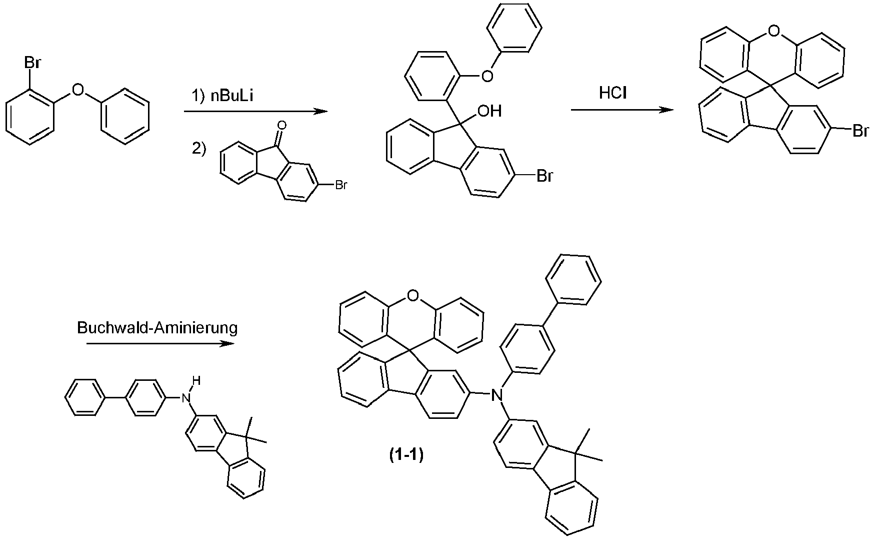

### Zwischenstufe: Brom-Spirofluoren-Xanthen-Derivat

31,7 g (127 mmol) 1-Bromo-2-diphenylether werden in einem ausgeheizten Kolben in 400 mL getrocknetem THF gelöst. Die Reaktionsmischung wird auf -78°C gekühlt. Bei dieser Temperatur werden 55 mL einer 2,5M-Lösung n-BuLi in Hexan (127 mmol) langsam zugetropft. Der Ansatz wird 1 Stunde bei -70°C nachgerührt. Anschließend werden 30 g 2-Bromfluorenon (116 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt.

Die einrotierte Lösung wird vorsichtig mit 300 ml Essigsäure versetzt und anschließend werden 50 ml rauchende HCl zugegeben. Der Ansatz wird auf 75°C erhitzt und 6 Stunden dort gehalten. Dabei fällt ein weißer Feststoff aus. Der Ansatz wird nun auf Raumtemperatur abgekühlt und der ausgefallene Feststoff wird abgesaugt und mit Methanol nachgewaschen. Ausbeute: 45 g (95%)

Analog dazu werden folgende Verbindungen hergestellt:

| Edukt 1 | Edukt 2 | Produkt | Ausbeute |
|---|---|---|---|
| 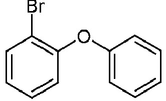 | 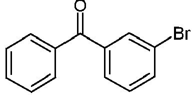 | 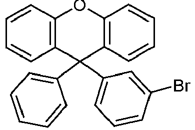 | 85% |
| 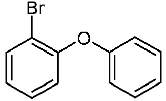 | 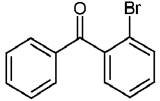 | 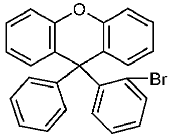 | 70% |
| 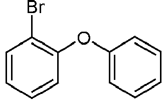 | 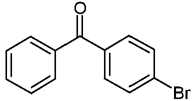 | 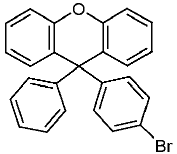 | 87% |
| 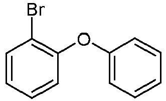 | 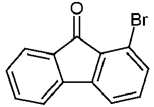 | 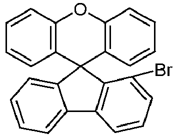 | 77% |
| 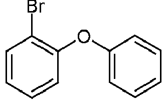 | 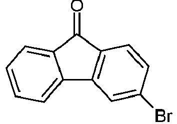 | 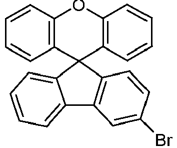 | 65% |
| 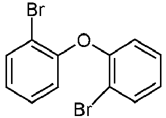 | 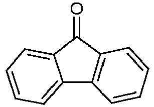 | 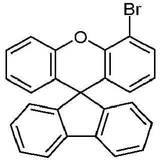 | 73% |
| 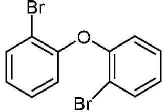 | 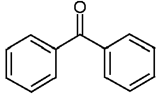 | 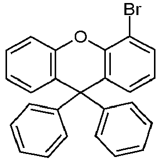 | 69% |
| 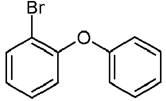 | 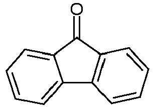 | 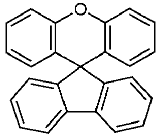 | 88% |
| 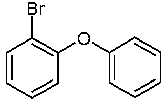 | 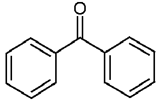 | 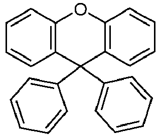 | 91% |
| 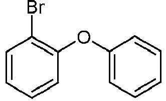 | 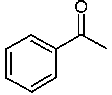 | 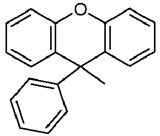 | 80% |
| 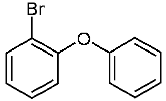 | 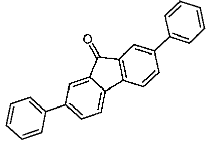 | 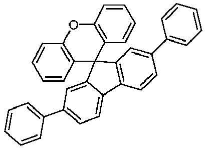 | 83% |

### Verbindung 1-1

17,6 g Biphenyl-4-yl-(9,9-dimethyl-9H-fluoren-2-yl)-amin (49 mmol) und 20,0 g des Brom-Spirofluoren-Xanthens (49 mmol) werden in 400 mL Toluol gelöst. Die Lösung wird entgast und mit N₂ gesättigt. Danach wird sie mit 2,43 mL (2,43 mmol) einer Tri-tert-Butylphosphin 1M-Lösung und 0,27 g (1,21 mmol) Palladium(II)acetat versetzt und anschließend werden 14 g Natrium-tert-butylat (146 mmol) zugegeben. Die Reaktionsmischung wird 6 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 27 g (80% d. Th).

Analog dazu werden folgende Verbindungen hergestellt:

| | Edukt 1 | Edukt 2 | Produkt | Ausbeute |
|---|---|---|---|---|
| **1-2#** | 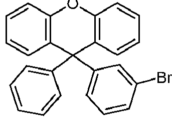 | 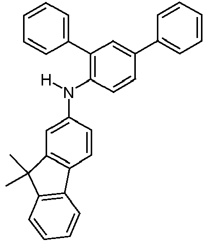 | 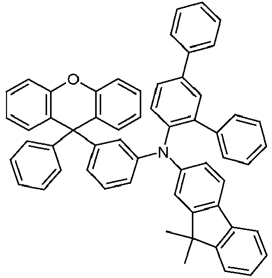 | 78 % |
| **1-3#** | 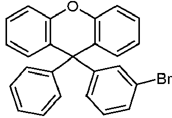 | 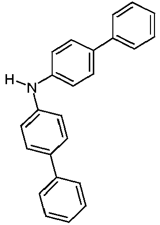 | 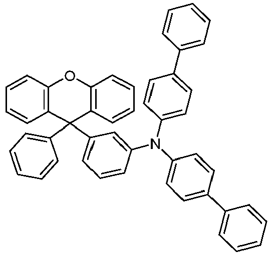 | 83% |
| **1-4#** | 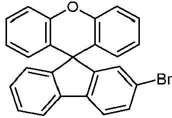 | 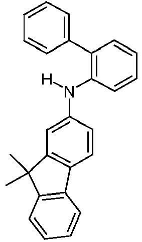 | 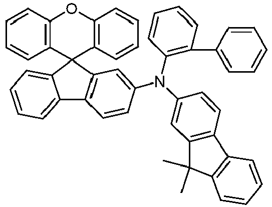 | 80% |
| **1-5#** | 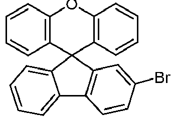 | 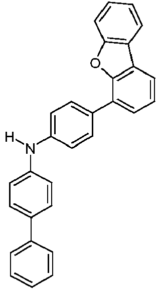 | 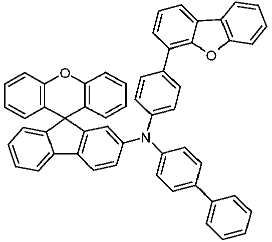 | 77% |
| **1-6#** | 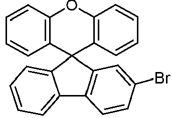 | 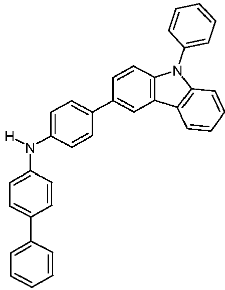 | 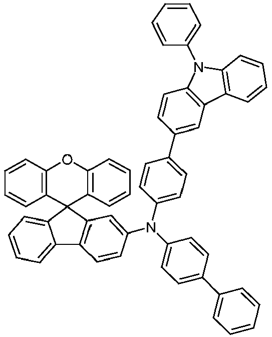 | 89% |
| **1-7#** | 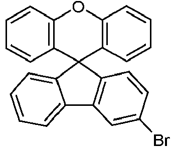 | 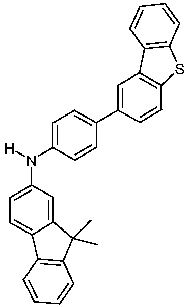 | 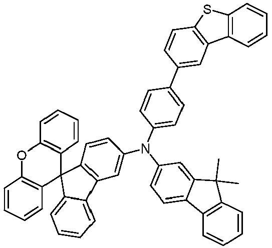 | 65% |
| **1-8#** | 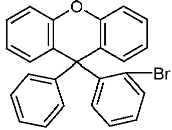 | 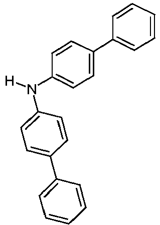 | 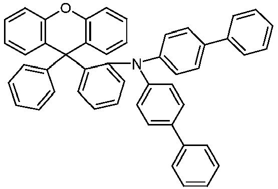 | 64% |
| **1-9#** | 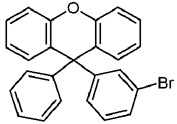 | 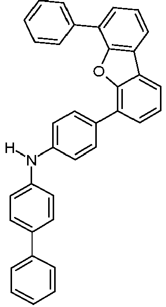 | 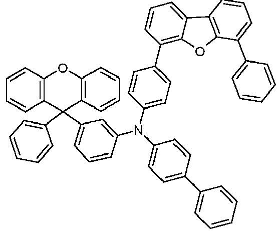 | 71% |
| **1-10#** | 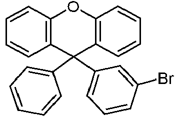 | 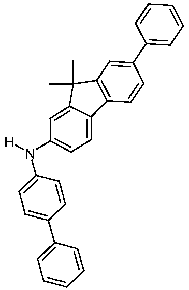 | 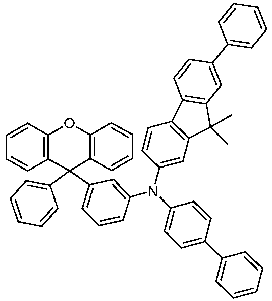 | 78% |
| **1-11#** | 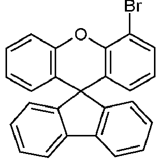 | 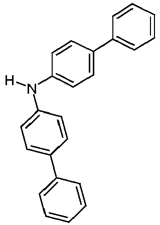 | 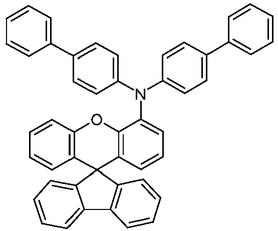 | 83% |
| **1-12#** | 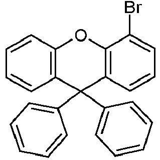 | 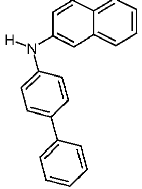 | 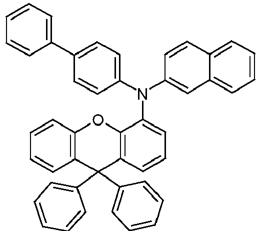 | 85% |

### Beispiel 2-1 #:

### Synthese der erfindungsgemäßen Verbindung 2-1

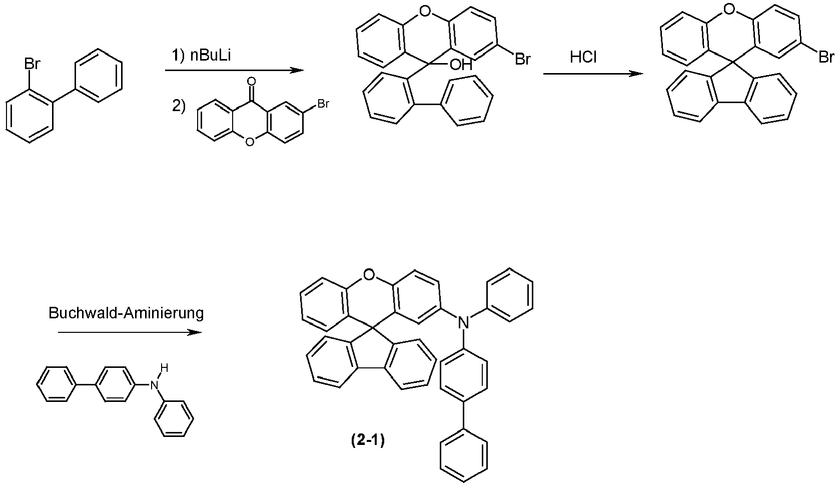

### Zwischenstufe: Brom-Spirofluoren-Xanthen-Derivat

30 g (129 mmol) 2-Brom-Biphenyl werden in einem ausgeheizten Kolben in 500 mL getrocknetem THF gelöst. Die Reaktionsmischung wird auf -78°C gekühlt. Bei dieser Temperatur werden 57 mL einer 2,5M-Lösung n-BuLi in Hexan (142 mmol) langsam zugetropft. Der Ansatz wird 1 Stunde bei - 70°C nachgerührt. Anschließend werden 35,4 g 2-Brom-Xanthen-9-on (129 mmol) in 150 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt.

Die einrotierte Lösung wird vorsichtig mit 300 ml Essigsäure versetzt und anschließend werden 50 ml rauchende HCl zugegeben. Der Ansatz wird auf 75°C erhitzt und 6 Stunden dort gehalten. Dabei fällt ein weißer Feststoff aus. Der Ansatz wird nun auf Raumtemperatur abgekühlt und der ausgefallene Feststoff wird abgesaugt und mit Methanol nachgewaschen. Ausbeute: 31.5 g (60%)

Analog dazu werden folgende Verbindungen hergestellt:

| Edukt 1 | Edukt 2 | Produkt | Ausbeute |
|---|---|---|---|
| 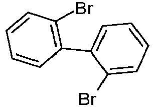 | 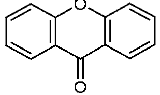 | 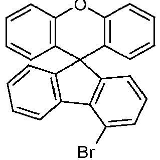 | 78 % |
| 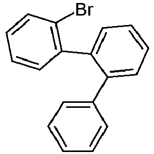 | 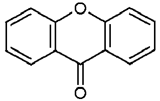 | 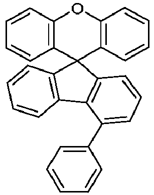 | 75% |
| 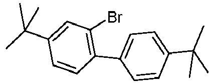 | 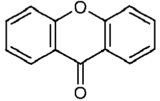 | 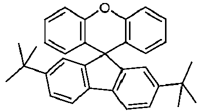 | 75% |

### Verbindung 2-1

Diese Verbindung wird analog zu Verbindung 1-1 hergestellt.
Ausbeute: 78%. Reinheit 99.9%

Analog dazu werden folgende Verbindungen hergestellt:

| | Edukt 1 | Edukt 2 | Produkt | Ausbeute |
|---|---|---|---|---|
| **2-2#** | 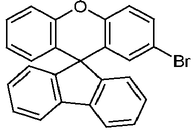 | 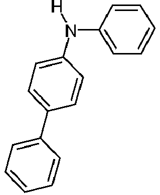 | 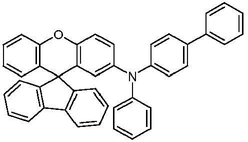 | 78% |
| **2-3#** | 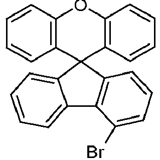 | 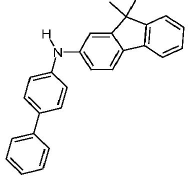 | 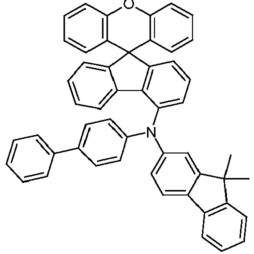 | 72% |
| **2-4#** | 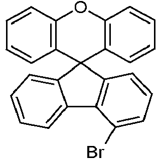 | 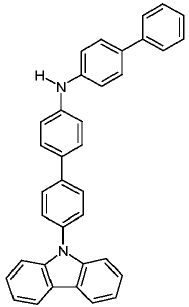 | 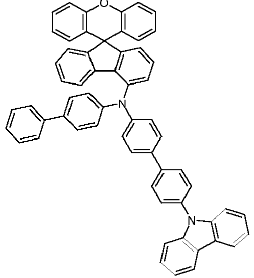 | 75% |
| **2-5#** | 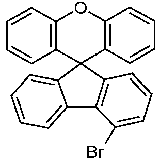 | 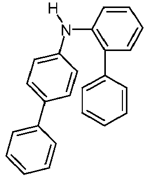 | 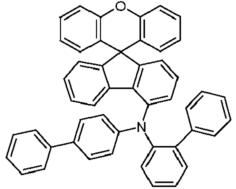 | 82% |
| **2-6#** | 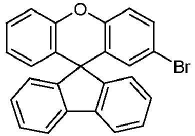 | 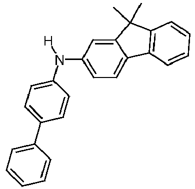 | 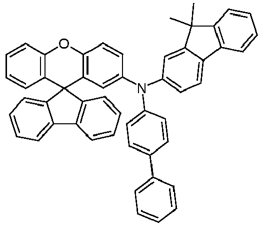 | 79% |

### Beispiel 3-1 #:

### Synthese der erfindungsgemäßen Verbindung 3-1

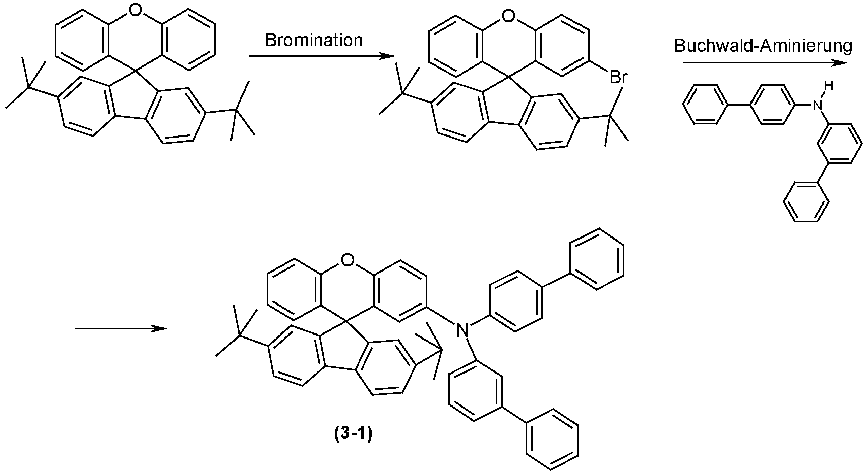

### Zwischenstufe: Brom-Spirofluoren-Xanthen-Derivat

20 g (60 mmol) Spirofluoren-Xanthen werden in 300 mL Acetonitril vorgelegt. Anschließend tropft man unter Lichtausschluss bei 0 °C eine Lösung aus 10,7 g (60 mmol) NBS in 50 ml CH₃CN hinzu, lässt auf RT kommen und rührt 4 h weiter bei 50°C. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 13,8 g, 55,9 % d. Th., Reinheit nach ¹H-NMR ca. 97 %.

Analog dazu werden folgende Verbindungen hergestellt:

| Edukt 1 | Produkt | Ausbeute |
|---|---|---|
| 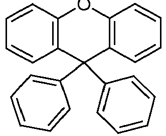 | 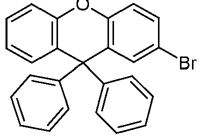 | 65% |
| 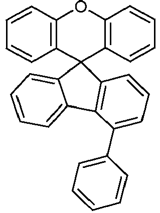 | 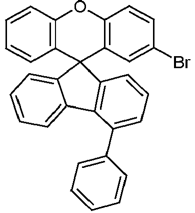 | 67% |
| 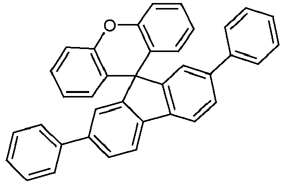 | 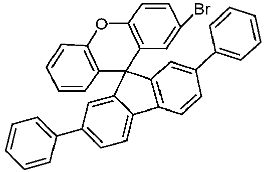 | 71% |
| 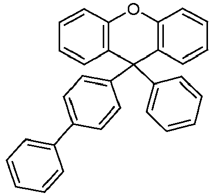 | 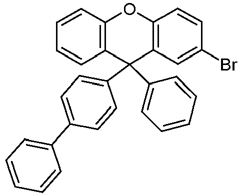 | 67% |
| 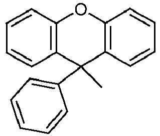 | 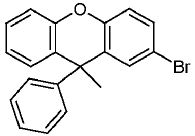 | 62% |

### Verbindung 3-1

Diese Verbindung wird analog zu Verbindung 1-1 hergestellt.
Ausbeute: 81%. Reinheit 99.9%

Analog dazu werden folgende Verbindungen hergestellt:

| | Edukt 1 | Edukt 2 | Produkt | Ausbeute |
|---|---|---|---|---|
| 3-2 # | 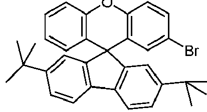 | 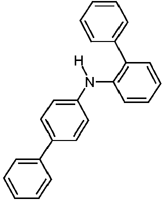 | 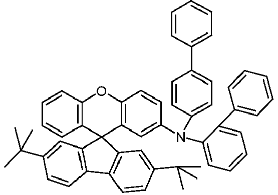 | 81% |
| **3-3#** | 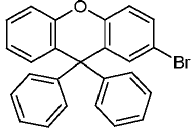 | 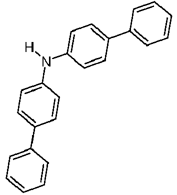 | 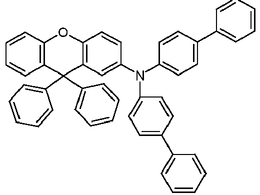 | 78 % |
| **3-4#** | 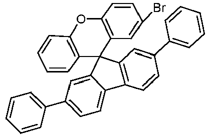 | 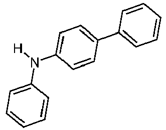 | 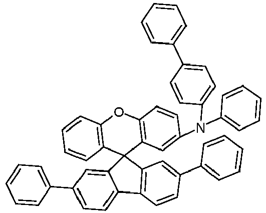 | 66% |
| **3-5#** | 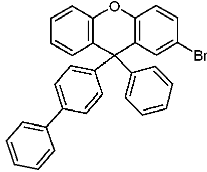 | 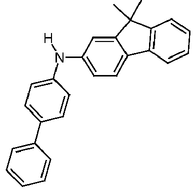 | 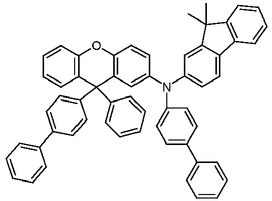 | 77% |
| **3-6#** | 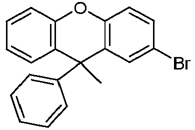 | 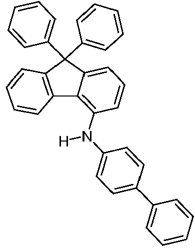 | 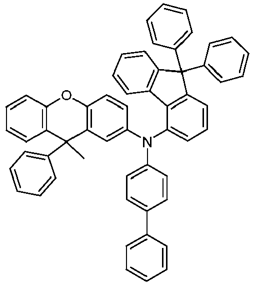 | 75% |
| **3-7#** | 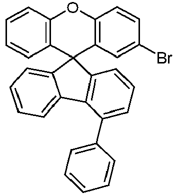 | 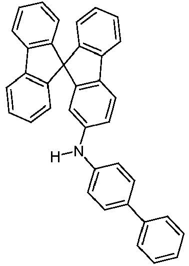 | 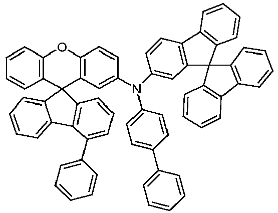 | 92% |

### Beispiel 4-1 #:

### Synthese der erfindungsgemäßen Verbindung 4-1

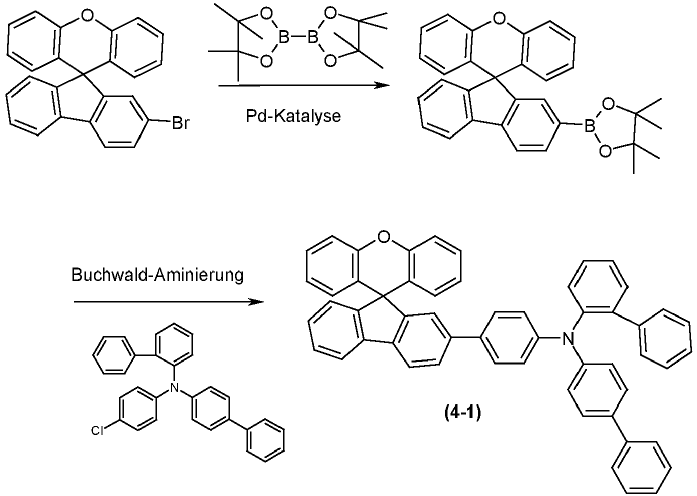

### Zwischenstufe: Spirofluoren-Xanthenboronester-Derivat

20 g (49 mmol) des Spirofluorenxanthen-Bromderivats,13,6 g (53 mmol) Bis(pinacolato)diboran und 14,3 g (146 mmol) Kalliumacetat werden in 500 ml Dioxan suspendiert. Zu dieser Suspension werden 1,19 g (1 mmol) 1,1-Bis(diphenylphosphino)ferrocen-dichloropalladium(II) Komplex mit DCM gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird aus Toluol umkristallisiert (20 g, 90% Ausbeute).

Analog dazu werden folgende Verbindungen hergestellt:

| Edukt 1 | Produkt | Ausbeute |
|---|---|---|
| 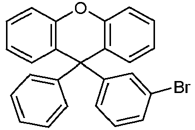 | 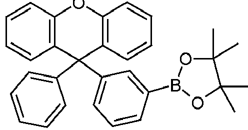 | 90% |
| 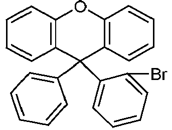 | 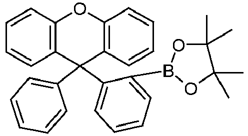 | 80% |
| 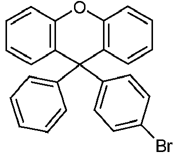 | 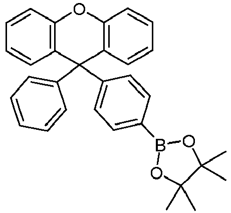 | 88% |
| 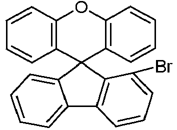 | 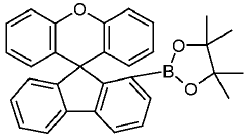 | 82% |
| 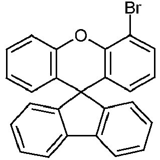 | 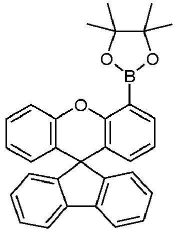 | 89% |
| 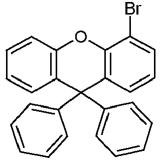 | 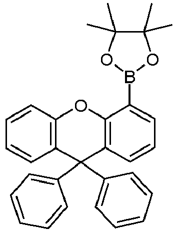 | 84% |
| 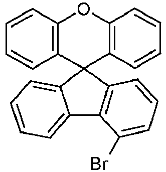 | 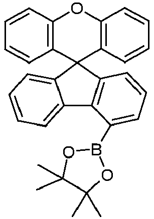 | 80% |

### Vorstufe: Biphenyl-2-yl-biphenyl-4-yl-(4-chloro-phenyl)-amin

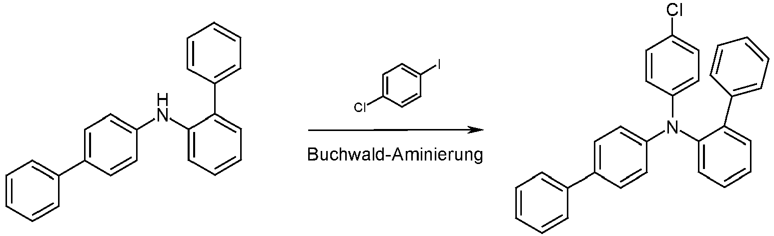

23,8 g Biphenyl-2-yl-biphenyl-4-yl-amin (74 mmol) und 21,2 g 4-Chloriodbenzol-Bromofluoren (89 mmol) werden in 500 mL Toluol gelöst: Die Lösung wird entgast und mit N₂ gesättigt. Danach wird sie mit 3 mL (3 mmol) einer Tri-tert-Butylphosphin 1M-Lösung und 0,33 g (1,48 mmol) Palladium(II)acetat versetzt und anschließend werden 10,7 g Natrium-tert-butylat (111 mmol) zugegeben. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 29 g (90% d. Th).

Analog dazu werden folgende Verbindungen hergestellt:

| Edukt 1 | Edukt 2 | Produkt | Ausbeute |
|---|---|---|---|
| 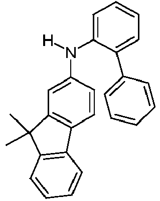 | 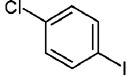 | 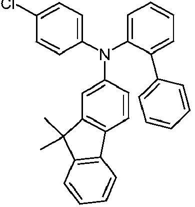 | 78 % |
| 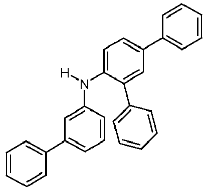 | 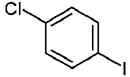 | 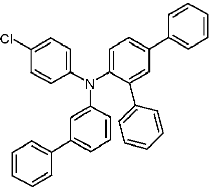 | 80% |
| 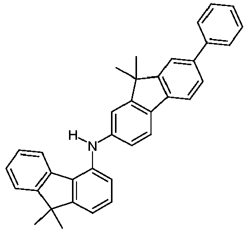 | 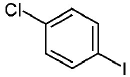 | 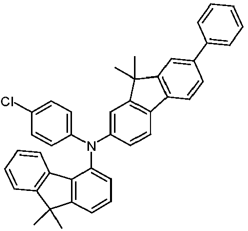 | 67% |
| 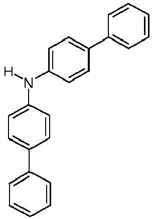 | 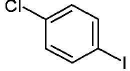 | 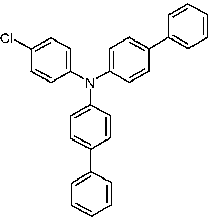 | 81% |
| 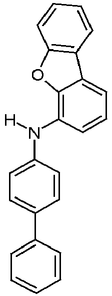 | 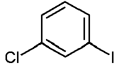 | 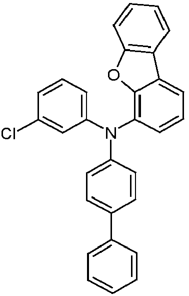 | 92% |
| 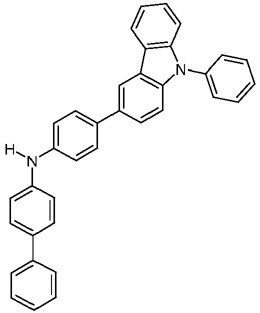 | 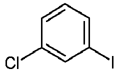 | 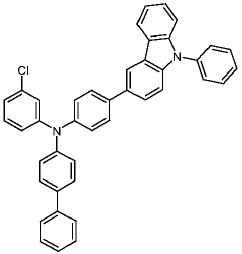 | 85% |
| 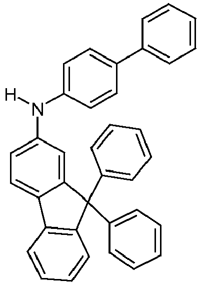 | 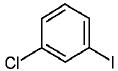 | 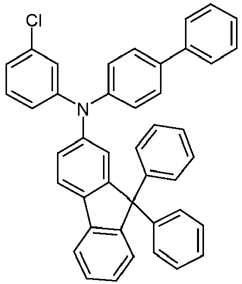 | 80% |
| 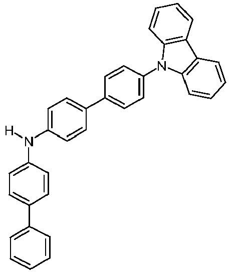 | 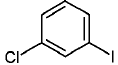 | 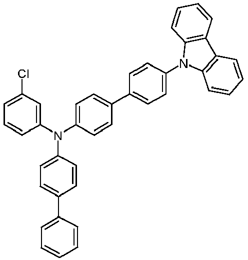 | 75% |
| 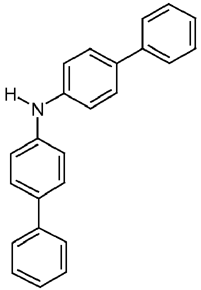 | 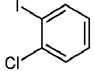 | 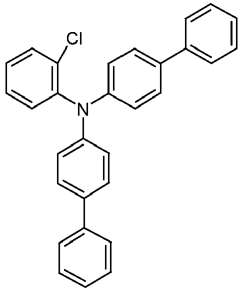 | 75% |

### Verbindung 4-1

13,8 g (30 mmol) Spirofluoren-Xanthen-pinacolboronester, 13 g (30 mmol) 2,7-Dibromfluorenon werden in 300 mL Dioxan und 9,1 g Caesiumfluorid (60 mmol) suspendiert. Zu dieser Suspension werden 2,2 g (3 mmol) Palladium-dichlorid-bis(tricyclohexylphosphin) gegeben, und die Reaktionsmischung wird 18 h unter Rückfluss erhitzt. Nach Erkalten wird die organische Phase abgetrennt, über Kieselgel filtriert, dreimal mit 80 mL Wasser gewaschen und anschließend zur Trockene eingeengt. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 17,8 g (85% d. Th).

Analog dazu werden folgende Verbindungen hergestellt:

| | Edukt 1 | Edukt 2 | Produkt | Ausbeute |
|---|---|---|---|---|
| **4-2#** | 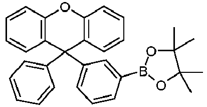 | 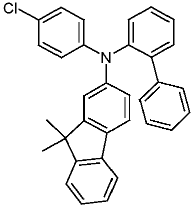 | 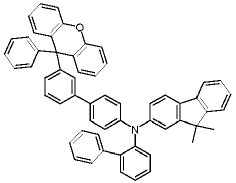 | 78 % |
| **4-3#** | 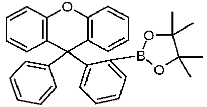 | 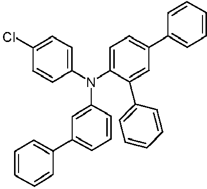 | 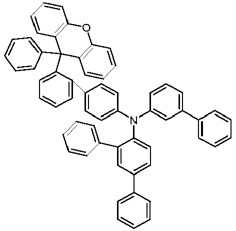 | 71% |
| **4-4#** | 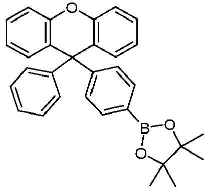 | 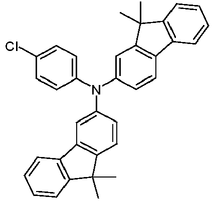 | 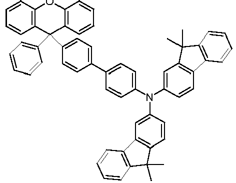 | 82% |
| **4-5#** | 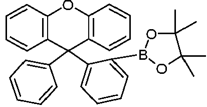 | 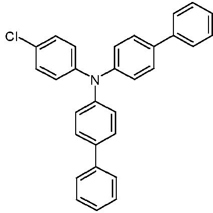 | 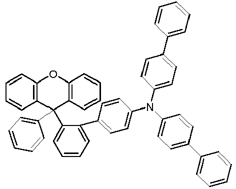 | 89% |
| **4-6#** | 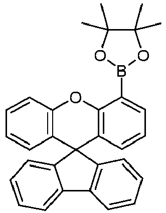 | 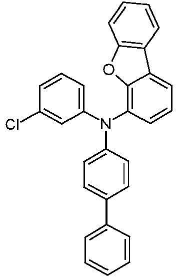 | 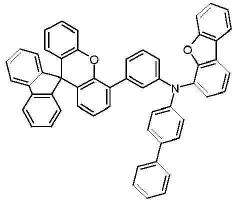 | 69% |
| **4-7#** | 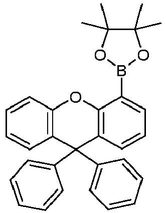 | 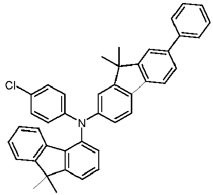 | 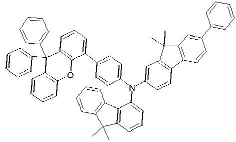 | 88% |
| **4-8#** | 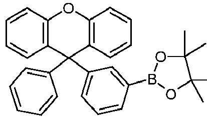 | 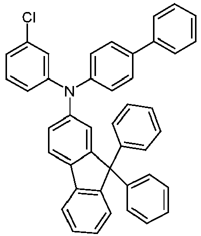 | 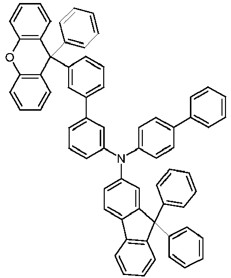 | 85% |
| **4-9#** | 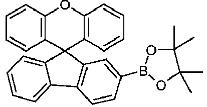 | 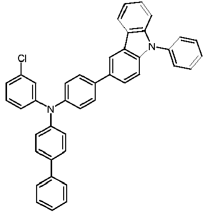 | 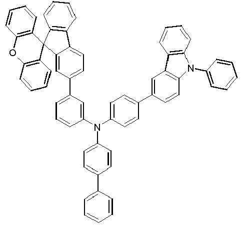 | 75% |
| **4-10#** | 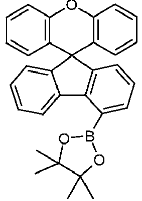 | 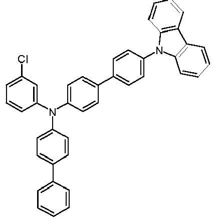 | 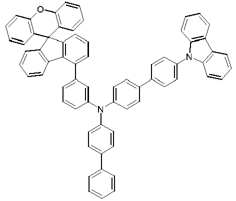 | 75% |
| **4-11#** | 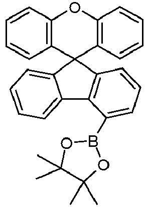 | 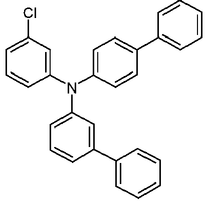 | 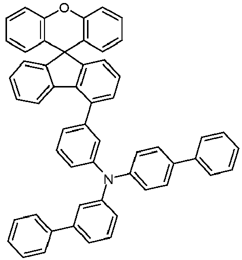 | 80% |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden erfinderischen Beispielen E1 bis E6 und in den Referenzbeispielen V1 bis V4 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht (HTL1) / Lochtransportschicht (HTL2) / p-dotierte Lochtransportschicht (HTL3) / Lochtransportschicht (HTL4) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, die verschiedenen Bauteilaufbauten in Tabelle 2

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1 (5%) bedeutet hierbei, dass das Material H1 in einem Volumenanteil von 95% und SEB1 in einem Anteil von 5% in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LD80 @ 50 mA/cm² ist die Lebensdauer, bis zu der die OLED bei einer Starthelligkeit bei konstantem Strom von 50mA/cm² auf 80 % der Anfangsintensität abgefallen ist.

| Tabelle 1: Strukturen der verwendeten Materialien | | | | | | | |
|---|---|---|---|---|---|---|---|
| 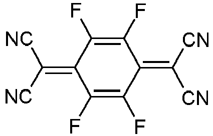 | 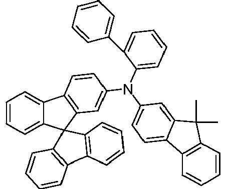 | 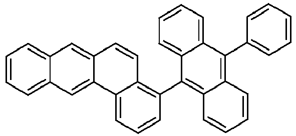 | | | | | |
| F4TCNQ | HIL | H1 | | | | | |
| 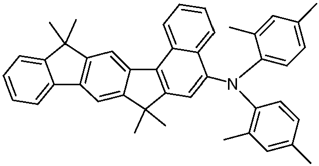 | 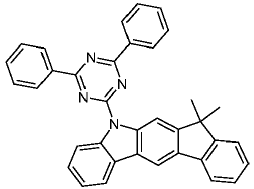 | 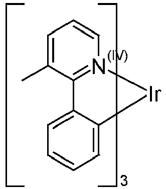 | | | | | |
| SEB | H2 | TEG | | | | | |
| 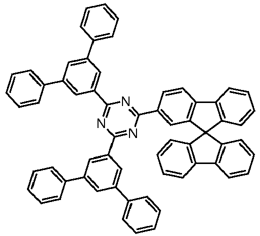 | 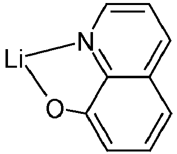 | | | | | | |
| ETM | LiQ | | | | | | |
| 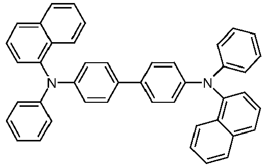 | 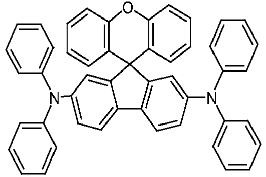 | 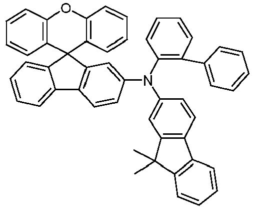 | | | | | |
| NPB | HTMV1 | HTM1 | | | | | |
| 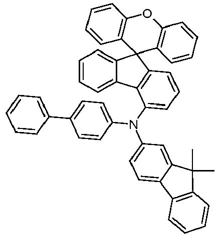 | 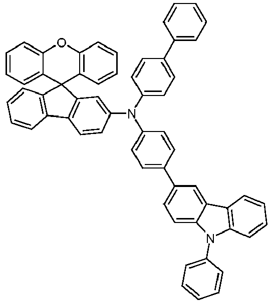 | 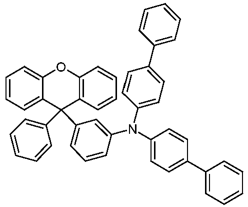 | | | | | |
| HTM2 | HTM3 | HTM4 | | | | | |

| Tabelle 2: Aufbau der OLEDs | | | | | | | |
|---|---|---|---|---|---|---|---|
| **Bsp** | **HTL1** | **HTL2** | **HTL3** | **HTL4** | **EML** | **ETL** | **EIL** |
| | Dicke / nm | Dicke / nm | Dicke / nm | Dicke / nm | Dicke / nm | Dicke / nm | Dicke / nm |
| V1 | HIM1: F4TCNQ(3%) | HIM1 | NPB: F4TCNQ(3%) | NPB | H1:SEB1 (5%) | ETM(50%):LiQ (50%) | LiQ |
| | 20 nm | 155 nm | 20 nm | 20 nm | 20 nm | 30 nm | 1 nm |
| V2 | HIM1: F4TCNQ(3%) | HIM1 | HTMV1 : F4TCNQ(3%) | HTMV1 | H1:SEB1(5%) | ETM(50%):LiQ (50%) | LiQ |
| | 20 nm | 155 nm | 20 nm | 20 nm | 20 nm | 30 nm | 1 nm |
| E1# | HIM1: F4TCNQ(3%) | HIM1 | HTM1: F4TCNQ(3%) | HTM1 | H1:SEB1(5%) | ETM(50%):LiQ (50%) | LiQ |
| | 20 nm | 155 nm | 20 nm | 20 nm | 20 nm | 30 nm | 1 nm |
| E2# | HIM1: F4TCNQ(3%) | HIM1 | HTM2: F4TCNQ(3%) | HTM2 | H1:SEB1(5%) | ETM(50%):LiQ(50%) | LiQ |
| | 20 nm | 155 nm | 20 nm | 20 nm | 20 nm | 30 nm | 1 nm |
| V3 | HIM1: F4TCNQ(3%) | HIM1 | NPB: F4TCNQ(3%) | NPB | H2:TEG(10%) | ETM(50%):LiQ (50%) | LiQ |
| | 20 nm | 210 nm | 20 nm | 20 nm | 30 nm | 40 nm | 1 nm |
| V4 | HIM1: F4TCNQ(3%) | HIM1 | HTMV1 : F4TCNQ(3%) | HTMV1 | H2:TEG(10%) | ETM(50%):LiQ (50%) | LiQ |
| | 20 nm | 210 nm | 20 nm | 20 nm | 30 nm | 40 nm | 1 nm |
| E3# | HIM1: F4TCNQ(3%) | HIM1 | HTM1: F4TCNQ(3%) | HTM1 | H2:TEG(10%) | ETM(50%):LiQ (50%) | LiQ |
| | 20 nm | 210 nm | 20 nm | 20 nm | 30 nm | 40 nm | 1 nm |
| E4# | HIM1: F4TCNQ(3%) | HIM1 | HTM2: F4TCNQ(3%) | HTM2 | H2:TEG(10%) | ETM(50%):LiQ (50%) | LiQ |
| | 20 nm | 210 nm | 20 nm | 20 nm | 30 nm | 40 nm | 1 nm |
| E5# | HIM1: F4TCNQ (3%) | HIM1 | HTM3: F4TCNQ (3%) | HTM3 | H1:SEB1(5%) | ETM(50%):LiQ (50%) | LiQ |
| | 20 nm | 155 nm | 20 nm | 20 nm | 20 nm | 30 nm | 1 nm |
| E6# | HIM1: F4TCNQ (3%) | HIM1 | HTM4: F4TCNQ (3%) | HTM4 | H2:TEG(10%) | ETM(50%):LiQ (50%) | LiQ |
| | 20 nm | 210 nm | 20 nm | 20 nm | 30 nm | 40 nm | 1 nm |

In den vorgestellten Beispielen E1 bis E6 werden die erfindungsgemäßen Verbindungen HTM1, HTM2, HTM3 und HTM4 in der p-dotierten Lochtransportschicht HTL3 und der undotierten Lochtransportschicht HTL4 eingesetzt. Dies verdeutlicht beispielhaft die Verwendung als lochtransportierendes Material. Es können alternativ andere p-Dotanden verwendet werden in Kombination mit den erfindungsgemäßen Verbindungen. Weiterhin alternativ können die erfindungsgemäßen Verbindungen in einer anderen lochtransportierenden Schicht oder in anderen Device-Aufbauten verwendet werden.

In den Vergleichsvorrichtungen V1 bis V4 sind statt der erfindungsgemäßen Verbindungen Lochtransportverbindungen aus dem Stand der Technik eingesetzt (Verbindungen NPB bzw. HTMV1).

### Beispiel 1 (fluoreszierende OLEDs) #

Hier zeigen im Vergleich zu den Referenzvorrichtungen V1 und V2 (Quanteneffizienz 6,2 % und 7,7 %) die beiden erfindungsgemäßen Vorrichtungen E1 und E2 mit 8,0 % und 8,4 % eine höhere Quanteneffizienz bei 10 mA/cm². Die Lebensdauer LT80 bei 50 mA/cm² ist bei den erfindungsgemäßen Vorrichtungen E1 (225 h) und E2 (285 h) auch deutlich besser als bei den Referenzvorrichtungen V1 (135 h) und V2 (30 h).

### Beispiel 2 (phosphoreszierende OLEDs) #

Hier zeigen die Referenzvorrichtungen V3 und V4 (Quanteneffizienz 11,7 % bzw. 19,8 %) niedrigere bzw. annähernd gleiche Quanteneffizienzen bei 2 mA/cm² wie die erfindungsgemäßen Vorrichtungen E3 (Quanteneffizienz 20,4 %) und E4 (Quanteneffizienz 19,9 %). Auch sind die Lebensdauern bei 20 mA/cm² bei den erfindungsgemäßen Vorrichtungen E3 (160 h) und E4 (215 h) größer als bei den Vergleichs-Vorrichtungen V3 (80 h) und V4 (140 h).

### Beispiel 3 (fluoreszierende OLED) #

Hier zeigt die erfindunggemäße OLED E5 im Vergleich zu den Referenzvorrichtungen V1 und V2 (6,2 % und 7,7 %) mit 7,5 % ähnlich hohe bzw. höhere Quanteneffizienz bei 10 mA/cm². Die Lebensdauer LT80 bei 50 mA/cm² ist bei der erfindungsgemäßen OLED E5 (145 h) besser als bei den Referenzvorrichtungen V1 (135 h) und V2 (30 h).

### Beispiel 4 (phosphoreszierende OLED) #

Hier zeigt die erfindungsgemäße Vorrichtung E6 (19.2 %) eine höhere Quanteneffizienz bei 2mA/cm² als die Referenzvorrichtung V3 (11,7 %).

Zusammenfassend zeigen die Beispiele die sehr guten Device-Daten, welche bei Verwendung der erfindungsgemäßen Verbindungen als lochtransportierende Materialien in OLEDs erhalten werden. Weiterhin zeigen die Beispiele verbesserte Device-Daten verglichen mit Materialien gemäß dem Stand der Technik.

## Patentansprüche

1. Verbindung einer Formel (I) 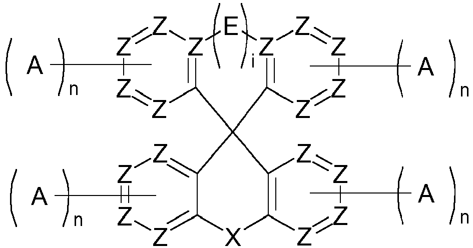 Formel (I), wobei gilt:
A ist eine Gruppe der folgenden Formel (A-II) 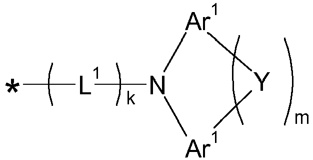 Formel (A-II), wobei gilt:
L¹ ist bei jedem Auftreten gleich oder verschieden ein aromatisches oder heteroaromatisches Ringsystem mit 6 bis 24 aromatischen Ringatomen, welches durch einen oder mehrere Reste R¹ substituiert sein kann;
Ar¹ ist bei jedem Auftreten gleich oder verschieden ein aromatisches oder heteroaromatisches Ringsystem mit 6 bis 30 aromatischen Ringatomen, das mit einem oder mehreren Resten R¹ substituiert sein kann;
Y ist eine Einfachbindung;
k ist gleich 0, 1, 2 oder 3;
m ist gleich 1;
wobei die Gruppe A über die mit * markierte Bindung an den Rest der Verbindung der Formel (I) gebunden ist;
E ist eine Einfachbindung;
X ist O oder S;
Z ist CR², wobei in dem Fall, dass eine Gruppe A an sie gebunden ist, die Gruppe Z gleich C ist;
R¹ ist bei jedem Auftreten gleich oder verschieden H, D, F, Cl, Br, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ring-atomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R¹ miteinander verknüpft sein können und einen Ring bilden können;
R² ist bei jedem Auftreten gleich oder verschieden H, D, F, Cl, Br, I, C(=O)R³, CN, Si(R³)₃, P(=O)(R³)², S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=NR³, -C(=O)O-, -C(=O)NR³-, P(=O)(R³), -O-, -S-, SO oder SO₂ ersetzt sein können und wobei ein oder mehrere H-Atome in den oben genannten Gruppen durch D, F, Cl, Br, I oder CN ersetzt sein können, und wobei zwei oder mehr Reste R² miteinander verknüpft sein können und einen Ring bilden können;
R³ ist bei jedem Auftreten gleich oder verschieden H, D, F, Cl, Br, I, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I oder CN ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
i ist gleich 0 oder 1;
n ist bei jedem Auftreten gleich oder verschieden 0 oder 1, wobei die Summe aller Indices n gleich 1 ist.

2. Verbindung nach Anspruch 1, **dadurch gekennzeichnet, dass** i gleich 1 ist.

3. Verbindung nach Anspruch 1 oder 2, **dadurch gekennzeichnet, dass** k gleich 0 ist.

4. Verbindung nach einem oder mehreren der Ansprüche 1 bis 3, **dadurch gekennzeichnet, dass** L¹ bei jedem Auftreten gleich oder verschieden gewählt ist aus Phenyl, Biphenyl, Naphthyl, Terphenyl, Fluorenyl, Spirobifluoren, Indenofluorenyl, Carbazol, Dibenzofuran, und Dibenzothiophen, die jeweils mit einem oder mehreren Resten R¹ substituiert sein können.

5. Verbindung nach einem oder mehreren der Ansprüche 1 bis 4, **dadurch gekennzeichnet, dass** X gleich O ist.

6. Verbindung nach einem oder mehreren der Ansprüche 1 bis 5, **dadurch gekennzeichnet, dass** die Verbindung der Formel (I) einer der Formeln (I-1) bis (I-8) entspricht 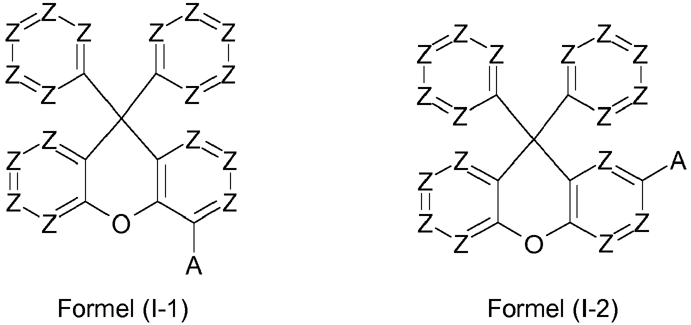 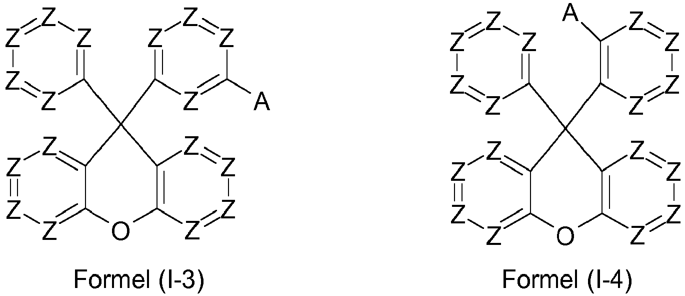 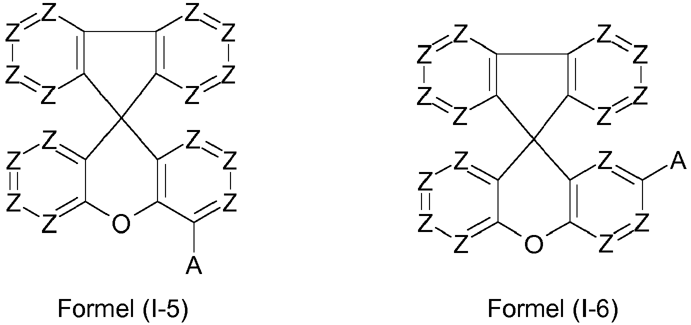 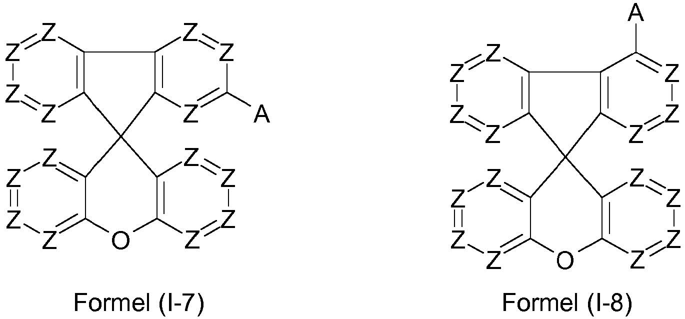 wobei die auftretenden Symbole wie in einem oder mehreren der Ansprüche 1 bis 5 definiert sind.

7. Verbindung nach einem oder mehreren der Ansprüche 1 bis 6, **dadurch gekennzeichnet, dass** die Gruppe A gewählt ist aus den folgenden Formeln:
| | |
|---|---|
| 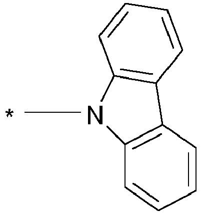 | 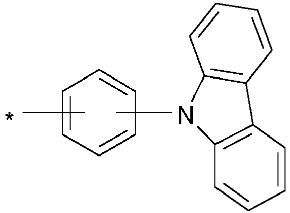 |
| Formel (A-II-19) | Formel (A-II-20) |
wobei alle freien Positionen in den Formeln mit einem oder mehreren Resten R¹ substituiert sein können.

8. Verbindung nach einem oder mehreren der Ansprüche 1 bis 7, **dadurch gekennzeichnet, dass**
R¹ bei jedem Auftreten gleich oder verschieden gewählt ist aus H, D, F, CN, Si(R³)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, -R³C=CR³-, Si(R³)₂, C=O, C=NR³, -NR³-, -O-, - S-, -C(=O)O- oder -C(=O)NR³- ersetzt sein können, und aromatischen oder heteroaromatischen Ringsystemen mit 5 bis 20 aromatischen Ringatomen, die jeweils mit einem oder mehreren Resten R³ substituiert sein können; und
R² bei jedem Auftreten gleich oder verschieden gewählt ist aus H, D, F, CN, Si(R³)₃, geradkettigen Alkyl- oder Alkoxygruppen mit 1 bis 20 C-Atomen, und verzweigten oder cyclischen Alkyl- oder Alkoxygruppen mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, -R³C=CR³-, Si(R³)₂, C=O, C=NR³, -O-, -S-, - C(=O)O- oder -C(=O)NR³- ersetzt sein können; und
R³ bei jedem Auftreten gleich oder verschieden gewählt ist aus H, D, F, CN, Si(R⁴)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, C=NR⁴, -NR⁴-, -O-, - S-, -C(=O)O- oder -C(=O)NR⁴- ersetzt sein können, und aromatischen oder heteroaromatischen Ringsystemen mit 5 bis 20 aromatischen Ringatomen, die jeweils mit einem oder mehreren Resten R⁴ substituiert sein können; und
R⁴ bei jedem Auftreten gleich oder verschieden gewählt ist aus H, D, F, und aliphatischen, aromatischen oder heteroaromatischen organischen Resten mit 1 bis 20 C-Atomen, in denen auch ein oder mehrere H-Atome durch D oder F ersetzt sein können.

9. Verfahren zur Herstellung einer Verbindung der Formel (I) nach einem oder mehreren der Ansprüche 1 bis 8, **dadurch gekennzeichnet, dass** der Grundkörper durch Addition eines metallorganischen Nukleophils an eine Carbonylgruppe hergestellt wird.

10. Oligomere, Polymere oder Dendrimere enthaltend eine oder mehrere Verbindungen gemäß Formel (I) nach einem oder mehreren der Ansprüche 1 bis 8, wobei die Bindung(en) zum Polymer, Oligomer oder Dendrimer an beliebigen, in Formel (I) mit R¹ oder R² substituierten Positionen lokalisiert sein können.

11. Formulierung, enthaltend mindestens eine Verbindung gemäß Formel (I) nach einem oder mehreren der Ansprüche 1 bis 8 oder mindestens ein Polymer, Oligomer oder Dendrimer nach Anspruch 10 sowie mindestens ein Lösungsmittel.

12. Verwendung einer Verbindung gemäß Formel (I) nach einem oder mehreren der Ansprüche 1 bis 8 in einer elektronischen Vorrichtung.

13. Elektronische Vorrichtung, enthaltend mindestens eine Verbindung gemäß Formel (I) nach einem oder mehreren der Ansprüche 1 bis 8.

14. Elektronische Vorrichtung nach Anspruch 13, **dadurch gekennzeichnet, dass** sie gewählt ist aus aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs).

15. Organische Elektrolumineszenzvorrichtung nach Anspruch 14, **dadurch gekennzeichnet, dass** die Verbindung gemäß Formel (I) in einer Lochtransportschicht, einer Elektronenblockierschicht, einer Lochinjektionsschicht oder in einer emittierenden Schicht enthalten ist.

## Claims

1. Compound of a formula (I) 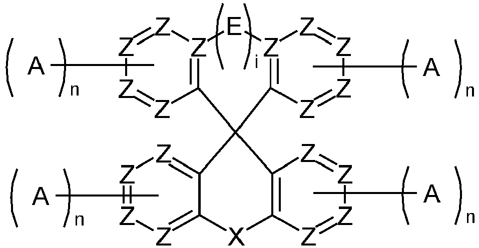 formula (I), where:
A is a group of the following formula (A-II) 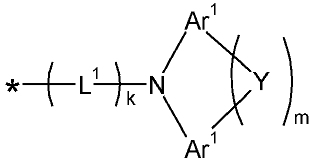 formula (A-II), where:
L¹ is on each occurrence, identically or differently, an aromatic or heteroaromatic ring system having 6 to 24 aromatic ring atoms, which may be substituted by one or more radicals R¹;
Ar¹ is on each occurrence, identically or differently, an aromatic or heteroaromatic ring system having 6 to 30 aromatic ring atoms, which may be substituted by one or more radicals R¹;
Y is a singel bond;
k is equal to 0, 1, 2 or 3;
m is equal to 1;
where the group A is bonded to the remainder of the compound of the formula (I) via the bond labelled with *;
E is a single bond;
X is O or S;
Z is CR², where, in the case where a group A is bonded to it, the group Z is equal to C;
R¹ is on each occurrence, identically or differently, H, D, F, CI, Br, I, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two or more radicals R¹ may be linked to one another and may form a ring;
R² is on each occurrence, identically or differently, H, D, F, Cl, Br, I, C(=O)R³, CN, Si(R³)₃, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C=C-, Si(R³)₂, C=O, C=NR³, -C(=O)O-, -C(=O)NR³-, P(=O)(R³), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, and where two or more radicals R² may be linked to one another and may form a ring;
R³ is on each occurrence, identically or differently, H, D, F, Cl, Br, I, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F, Cl, Br, I or CN, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ may be linked to one another and form a ring;
i is equal to 0 or 1;
n is on each occurrence, identically or differently, 0 or 1, where the sum of all indices n is equal to 1.

2. Compound according to Claim 1, **characterised in that** i is equal to 1.

3. Compound according to Claim 1 or 2, **characterised in that** k is equal to 0.

4. Compound according to one or more of Claims 1 to 3, **characterised in that** L¹ is selected on each occurrence, identically or differently, from phenyl, biphenyl, naphthyl, terphenyl, fluorenyl, spirobifluorene, indenofluorenyl, carbazole, dibenzofuran and dibenzothiophene, which may in each case be substituted by one or more radicals R¹.

5. Compound according to one or more of Claims 1 to 4, **characterised in that** X is equal to O.

6. Compound according to one or more of Claims 1 to 5, **characterised in that** the compound of the formula (I) conforms to one of the formulae (1-1) to (I-8) 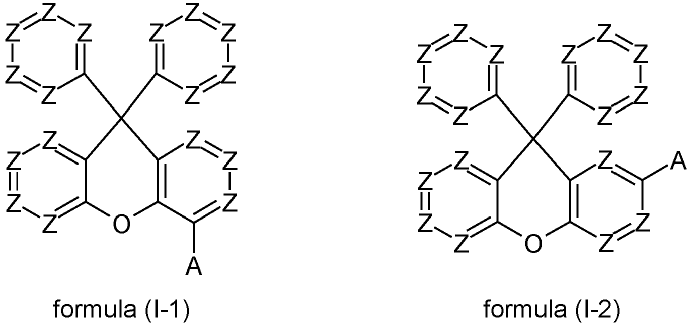 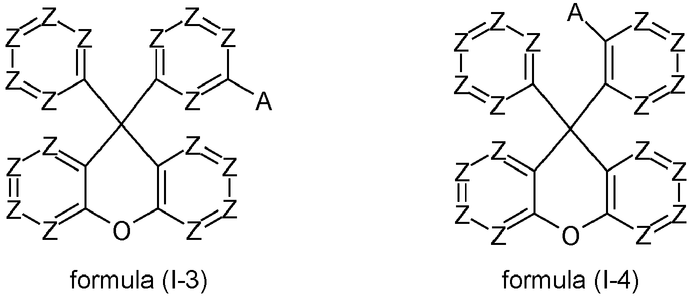 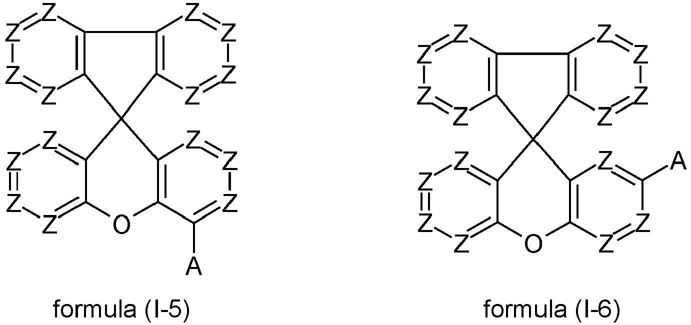 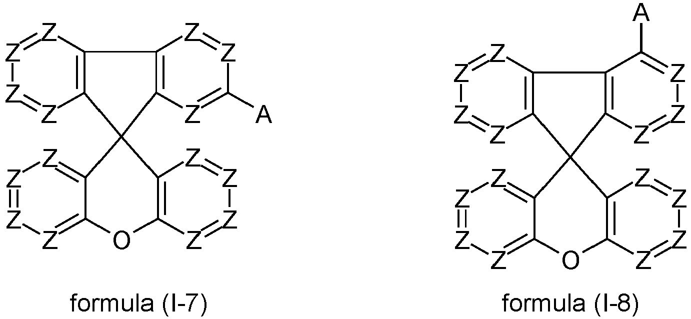 where the symbols occurring are as defined in one or more of Claims 1 to 5.

7. Compound according to one or more of Claims 1 to 6, **characterised in that** the group A is selected from the following formulae:
| | |
|---|---|
| 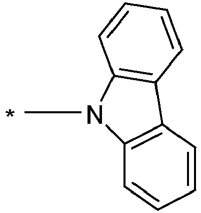 | 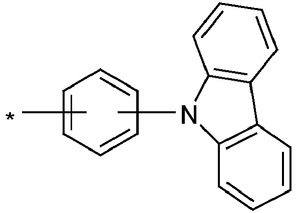 |
| formula (A-II-19) | formula (A-II-20) |
where all free positions in the formulae may be substituted by one or more radicals R¹.

8. Compound according to one or more of Claims 1 to 7, **characterised in that**
R¹ is selected on each occurrence, identically or differently, from H, D, F, CN, Si(R³)₃, straight-chain alkyl or alkoxy groups having 1 to 20 C atoms, branched or cyclic alkyl or alkoxy groups having 3 to 20 C atoms, where the above-mentioned groups may in each case be substituted by one or more radicals R³ and where one or more CH₂ groups in the above-mentioned groups may be replaced by -C=C-, -R³C=CR³-, Si(R³)₂, C=O, C=NR³, -NR³-, -O-, -S-, -C(=O)O- or -C(=O)NR³-, and aromatic or heteroaromatic ring systems having 5 to 20 aromatic ring atoms, which may in each case be substituted by one or more radicals R³; and
R² is selected on each occurrence, identically or differently, from H, D, F, CN, Si(R³)₃, straight-chain alkyl or alkoxy groups having 1 to 20 C atoms, and branched or cyclic alkyl or alkoxy groups having 3 to 20 C atoms, where the above-mentioned groups may in each case be substituted by one or more radicals R³ and where one or more CH₂ groups in the above-mentioned groups may be replaced by -C=C-, -R³C=CR³-, Si(R³)₂, C=O, C=NR³, -O-, -S-, -C(=O)O- or -C(=O)NR³⁻; and
R³ is selected on each occurrence, identically or differently, from H, D, F, CN, Si(R⁴)₃, straight-chain alkyl or alkoxy groups having 1 to 20 C atoms, branched or cyclic alkyl or alkoxy groups having 3 to 20 C atoms, where the above-mentioned groups may in each case be substituted by one or more radicals R⁴ and where one or more CH₂ groups in the above-mentioned groups may be replaced by -C=C-, -R⁴C=CR⁴-, Si(R⁴)₂, C=O, C=NR⁴, -NR⁴-, -O-, -S-, -C(=O)O- or -C(=O)NR⁴- ersetzt sein können, and aromatic or heteroaromatic ring systems having 5 to 20 aromatic ring atoms, which may in each case be substituted by one or more radicals R⁴; and
R⁴ is selected on each occurrence, identically or differently, from H, D, F, and aliphatic, aromatic or heteroaromatic organic radicals having 1 to 20 C atoms, in which, in addition, one or more H atoms may be replaced by D or F.

9. Process for the preparation of a compound of the formula (I) according to one or more of Claims 1 to 8, **characterised in that** the basic structure is prepared by addition of an organometallic nucleophile onto a carbonyl group.

10. Oligomers, polymers or dendrimers containing one or more compounds of the formula (I) according to one or more of Claims 1 to 8, where the bond(s) to the polymer, oligomer or dendrimer may be localised at any desired positions in formula (I) that are substituted by R¹ or R².

11. Formulation comprising at least one compound of the formula (I) according to one or more of Claims 1 to 8 or at least one polymer, oligomer or dendrimer according to Claim 10 and at least one solvent.

12. Use of a compound of the formula (I) according to one or more of Claims 1 to 8 in an electronic device.

13. Electronic device comprising at least one compound of the formula (I) according to one or more of Claims 1 to 8.

14. Electronic device according to Claim 13, **characterised in that** it is selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

15. Organic electroluminescent device according to Claim 14, **characterised in that** the compound of the formula (I) is present in a hole-transport layer, an electron-blocking layer, a hole-injection layer or in an emitting layer.

## Revendications

1. Composé d'une formule (I) 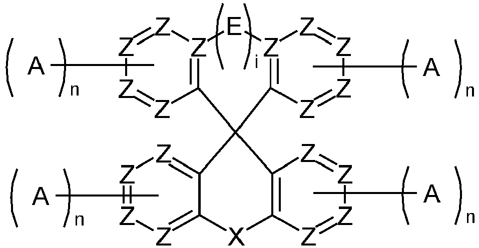 formule (I), dans laquelle :
A est un groupe de la formule (A-II) qui suit 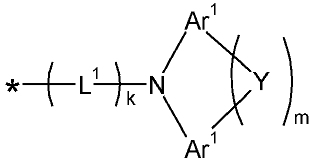 formule (A-II), dans laquelle :
L¹ est pour chaque occurrence, de manière identique ou différente, un système de cycle aromatique ou hétéroaromatique qui comporte de 6 à 24 atomes de cycle aromatique, lequel peut être substitué par un radical ou par plusieurs radicaux R¹;
Ar¹ est pour chaque occurrence, de manière identique ou différente, un système de cycle aromatique ou hétéroaromatique qui comporte de 6 à 30 atomes de cycle aromatique, lequel peut être substitué par un radical ou par plusieurs radicaux R¹ ;
Y est une liaison simple ;
k est égal à 0, 1, 2 ou 3 ;
m est égal à 1 ;
où le groupe A est lié au reste du composé de la formule (I) via la liaison qui est repérée à l'aide de * ;
E est une liaison simple ;
X est O ou S ;
Z est CR², où, dans le cas où un groupe A lui est lié, le groupe Z est égal à C ;
R¹ est pour chaque occurrence, de manière identique ou différente, H, D, F, Cl, Br, I, C(=O)R³, CN, Si(R³)₃, N(R³)₂, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C=C-, Si(R³)₂, C=O, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³, où deux radicaux R¹ ou plus peuvent être liés l'un à l'autre ou les uns aux autres et peuvent former un cycle ;
R² est pour chaque occurrence, de manière identique ou différente, H, D, F, Cl, Br, I, C(=O)R³, CN, Si(R³)₃, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, et où deux radicaux R² ou plus peuvent être liés l'un à l'autre ou les uns aux autres et peuvent former un cycle ;
R³ est pour chaque occurrence, de manière identique ou différente, H, D, F, Cl, Br, I, C(=O)R⁴, CN, Si(R⁴)₃, N(R⁴)₂, P(=O)(R⁴)₂, S(=O)R⁴, S(=O)₂R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂ et où un ou plusieurs atome(s) de H dans les groupes qui ont été mentionnés ci-avant peut/peuvent être remplacé(s) par D, F, Cl, Br, I ou CN, ou un système de cycle aromatique ou hétéroaromatique qui comporte de 5 à 30 atomes de cycle aromatique, lequel peut dans chaque cas être substitué par un radical ou par plusieurs radicaux R⁴, ou un groupe aryloxy ou hétéroaryloxy qui comporte de 5 à 30 atomes de cycle aromatique, lequel peut être substitué par un radical ou par plusieurs radicaux R⁴, où deux radicaux R³ ou plus peuvent être liés l'un à l'autre ou les uns aux autres et peuvent former un cycle ;
R⁴ est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/ peuvent être remplacé(s) par D ou par F ; deux substituants R⁴ ou plus peuvent être liés l'un à l'autre ou les uns aux autres et peuvent former un cycle ;
i est égal à 0 ou 1 ;
n est pour chaque occurrence, de manière identique ou différente, 0 ou 1, où la somme de tous les indices n est égale à 1.

2. Composé selon la revendication 1, **caractérisé en ce que** i est égal à 1.

3. Composé selon la revendication 1 ou 2, **caractérisé en ce que** k est égal à 0.

4. Composé selon une ou plusieurs des revendications 1 à 3, **caractérisé en ce que** L¹ est sélectionné pour chaque occurrence, de manière identique ou différente, parmi phényle, biphényle, naphtyle, terphényle, fluorényle, spirobifluorène, indénofluorényle, carbazole, dibenzofurane et dibenzothiophène, lequel peut dans chaque cas être substitué par un radical ou par plusieurs radicaux R¹.

5. Composé selon une ou plusieurs des revendications 1 à 4, **caractérisé en ce que** X est égal à O.

6. Composé selon une ou plusieurs des revendications 1 à 5, **caractérisé en ce que** le composé de la formule (I) est conforme à l'une des formules (I-1) à (I-8) 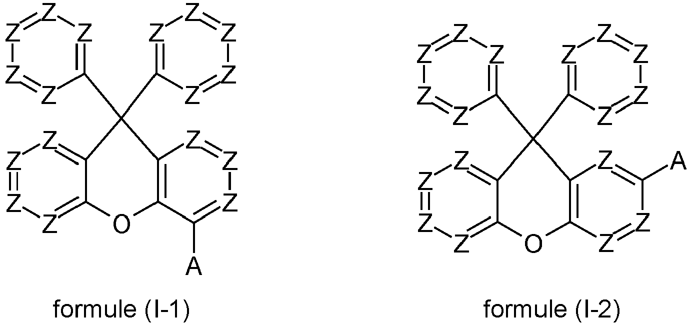 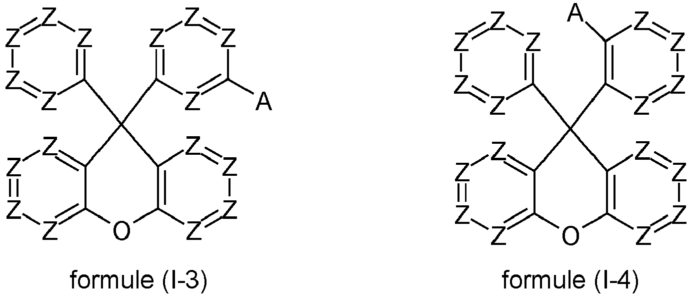 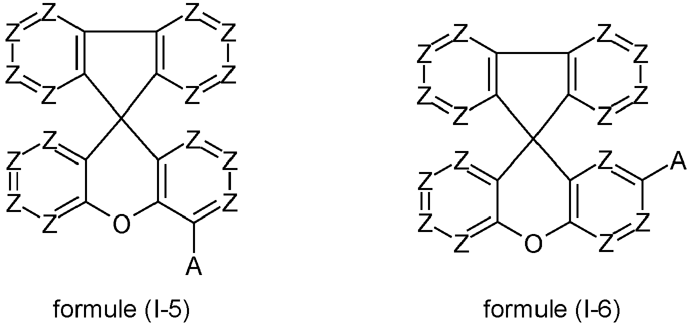 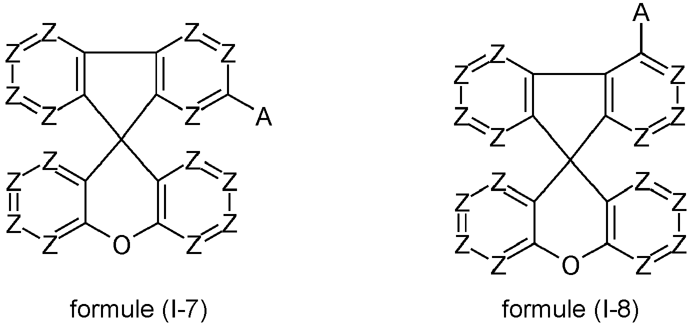 dans lesquelles les symboles qui sont présents sont tels que définis selon une ou plusieurs des revendications 1 à 5.

7. Composé selon une ou plusieurs des revendications 1 à 6, **caractérisé en ce que** le groupe A est sélectionné parmi les formules qui suivent :
| | |
|---|---|
| 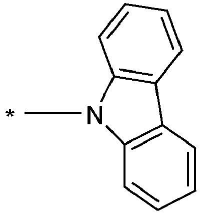 | 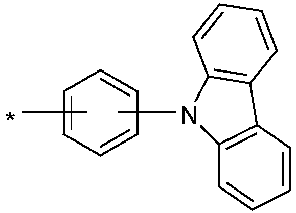 |
| formule (A-II-19) | formule (A-II-20) |
dans lesquelles toutes les positions libres dans les formules peuvent être substituées par un radical ou par plusieurs radicaux R¹.

8. Composé selon une ou plusieurs des revendications 1 à 7, **caractérisé en ce que**
R¹ est sélectionné pour chaque occurrence, de manière identique ou différente, parmi H, D, F, CN, Si(R³)₃, les groupes alkyle ou alcoxy en chaîne droite qui comportent de 1 à 20 atome(s) de C, les groupes alkyle ou alcoxy ramifiés ou cycliques qui comportent de 3 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -C≡C-, -R³C=CR³-, Si(R³)₂, C=O, C=NR3, -NR³-, -O-, -S-, -C(=O)O- ou -C(=O)NR³-, et les systèmes de cycle aromatique ou hétéroaromatique qui comportent de 5 à 20 atomes de cycle aromatique, lesquels peuvent dans chaque cas être substitués par un radical ou par plusieurs radicaux R³ ; et
R² est sélectionné pour chaque occurrence, de manière identique ou différente, parmi H, D, F, CN, Si(R³)₃, les groupes alkyle ou alcoxy en chaîne droite qui comportent de 1 à 20 atome(s) de C, et les groupes alkyle ou alcoxy ramifiés ou cycliques qui comportent de 3 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -C≡C-, -R³C=CR³-, Si(R³)₂, C=O, C=NR³, -O-, -S-, -C(=O)O- ou -C(=O)NR³-; et
R³ est sélectionné pour chaque occurrence, de manière identique ou différente, parmi H, D, F, CN, Si(R⁴)₃, les groupes alkyle ou alcoxy en chaîne droite qui comportent de 1 à 20 atome(s) de C, et les groupes alkyle ou alcoxy ramifiés ou cycliques qui comportent de 3 à 20 atomes de C, où les groupes qui ont été mentionnés ci-avant peuvent dans chaque cas être substitués par un radical ou par plusieurs radicaux R⁴ et où un ou plusieurs groupe(s) CH₂ dans les groupes qui ont été mentionnés ci-avant peut/peuvent être remplacé(s) par -C≡C-, -R⁴C=CR⁴-, Si(R⁴)₂, C=O, C=NR⁴, -NR⁴-, -O-, -S-, -C(=O)O- or -C(=O)NR⁴-, et les systèmes de cycle aromatique ou hétéroaromatique qui comportent de 5 à 20 atomes de cycle aromatique, lesquels peuvent dans chaque cas être substitués par un radical ou par plusieurs radicaux R⁴ ; et
R⁴ est sélectionné pour chaque occurrence, de manière identique ou différente, parmi H, D, F, et les radicaux organiques aliphatiques, aromatiques ou hétéroaromatiques qui comportent de 1 à 20 atome(s) de C, où, en outre, un ou plusieurs atome(s) de H peut/ peuvent être remplacé(s) par D ou par F.

9. Procédé pour la préparation d'un composé de la formule (I) selon une ou plusieurs des revendications 1 à 8, **caractérisé en ce que** la structure de base est préparée par ajout d'un nucléophile organométallique sur un groupe carbonyle.

10. Oligomères, polymères ou dendrimères contenant un ou plusieurs composé(s) de la formule (I) selon une ou plusieurs des revendications 1 à 8, dans lesquels la/les liaison(s) sur le polymère, sur l'oligomère ou sur le dendrimère peut/peuvent être localisée(s) au niveau de n'importe quelles positions souhaitées dans la formule (I) qui sont substituées par R¹ ou par R².

11. Formulation comprenant au moins un composé de la formule (I) selon une ou plusieurs des revendications 1 à 8 ou au moins un polymère, un oligomère ou un dendrimère selon la revendication 10 et au moins un solvant.

12. Utilisation d'un composé de la formule (I) selon une ou plusieurs des revendications 1 à 8 dans un dispositif électronique.

13. Dispositif électronique comprenant au moins un composé de la formule (I) selon une ou plusieurs des revendications 1 à 8.

14. Dispositif électronique selon la revendication 13, **caractérisé en ce qu'**il est sélectionné parmi le groupe qui est constitué par les circuits intégrés organiques (OIC), les transistors à effet de champ organiques (OFET), les transistors à film mince organiques (OTFT), les transistors à émission de lumière ou électroluminescents organiques (OLET), les cellules solaires organiques (OSC), les détecteurs optiques organiques, les photorécepteurs organiques, les dispositifs à extinction de champ organiques (OFQD), les cellules électrochimiques à émission de lumière ou électroluminescentes organiques (OLEC), les diodes laser organiques (O-laser) et les dispositifs électroluminescents organiques (OLED).

15. Dispositif électroluminescent organique selon la revendication 14, **caractérisé en ce que** le composé de la formule (I) est présent dans une couche de transport de trous, dans une couche de blocage d'électrons, dans une couche d'injection de trous ou dans une couche d'émission.
